# EUROPEAN PATENT APPLICATION

(11) **EP 4 844 377 A1**
(43) Date of publication of application: **25.11.2026**
(21) Application number: 25741743.6
(22) Date of filing: 08.01.2025
(51) Int. Cl.: G01N 21/359, C12M 1/34, C12Q 1/06, G01N 15/01

(54) **OBSERVATION DEVICE AND OBSERVATION METHOD**

(30) Priority: 17.01.2024 JP 2024005452
(71) Applicant: Sumitomo Electric Industries, Ltd., Osaka-shi, Osaka 541-0041 (JP); NATIONAL UNIVERSITY CORPORATION TOKAI NATIONAL HIGHER EDUCATION AND RESEARCH SYSTEM, Nagoya-shi, Aichi 464-8601 (JP)
(72) Inventor: SUGIYAMA Yoko, Osaka-shi, Osaka 541-0041 (JP); SUGANUMA Hiroshi, Osaka-shi, Osaka 541-0041 (JP); SANO Tomomi, Osaka-shi, Osaka 541-0041 (JP); KATO Ryuji, Nagoya-shi, Aichi 464-8601 (JP); TANAKA Kenjiro, Nagoya-shi, Aichi 464-8601 (JP)
(74) Representative: Boult Wade Tennant LLP
(86) International application number: PCT/JP2025/000378
(87) International publication number: WO 2025/154610

(57) **Abstract**

An observation device according to the present disclosure is for observing a state of a cell population configured of a plurality of cells. The observation device includes: at least one irradiation unit which is disposed at a position facing the cell population and irradiates at least one irradiation region set in the cell population with irradiation light having at least one wavelength included in the ranges of 650 nm to 850 nm and 1060 nm to 1090 nm; at least one light receiving unit which is disposed at a position facing the cell population and receives emission light emitted from the cell population as a result of irradiating the irradiation region with the irradiation light; and an analysis unit which is communicatively connected to the light receiving unit and determines a parameter including at least one of the number of cells and a cell density of the cell population in the irradiation region on the basis of an intensity of the emission light.

## Description

### Technical Field

The present disclosure relates to an observation device and an observation method. Priority is claimed on Japanese Patent Application No. 2024-005452, filed January 17, 2024, the content of which is incorporated herein by reference.

### Background Art

In recent years, for example, research has progressed on the application of planar or three-dimensional cell populations, called cell colonies, cell sheets, spheroids, or organoids, to drug discovery and regenerative medicine. One known method for non-invasive optical observation of a state of such a cell population is to observe an intensity of transmitted light from the cell population using a transmission microscope. Other known methods for non-invasive optical observation of a state of a cell population are, for example, methods disclosed in Patent Literature 1 to Patent Literature 4. Patent Literature 1 and Patent Literature 2 disclose methods for observing a distribution of intensities of transmitted or reflected light from a cell population. Patent Literature 3 and Patent Literature 4 disclose methods for observing an internal structure of a cell population using optical coherent tomography (OCT).

### Citation List

### Patent Literature

[Patent Literature 1] Japanese Unexamined Patent Publication No. 2018-004594
[Patent Literature 2] Japanese Unexamined Patent Publication No. 2020-094925
[Patent Literature 3] PCT International Publication No. WO 2015/004762
[Patent Literature 4] PCT International Publication No. WO 2017/216930

### Summary of Invention

An observation device of the present disclosure is an observation device configured to observe a state of a cell population configured of a plurality of cells. The observation device includes: at least one irradiation unit that is disposed at a position facing the cell population and irradiates at least one irradiation region set in the cell population with irradiation light having at least one wavelength included in the ranges from 650 nm to 850 nm and from 1060 nm to 1090 nm; at least one light receiving unit that is disposed at a position facing the cell population and receives emission light emitted from the cell population as a result of irradiating the irradiation region with the irradiation light; and an analysis unit that is communicatively connected to the light receiving unit and determines a parameter including at least one of the number of cells and a cell density of the cell population in the irradiation region on the basis of an intensity of the emission light.

### Brief Description of Drawings

Fig. 1 is a diagram showing a configuration of an observation device of a first embodiment.
Fig. 2 is an enlarged view of a part of the observation device of Fig. 1.
Fig. 3 is a diagram showing a hardware configuration of an analysis unit of Fig. 1.
Fig. 4 is a diagram showing a functional configuration of the analysis unit of Fig. 1.
Fig. 5 is a graph showing a spectrum of an amount of change in light intensity of cells.
Fig. 6 is a graph showing a spectrum of an amount of change in light intensity of a culture medium.
Fig. 7 is a flowchart showing an example of an observation method performed using the observation device of Fig. 1.
Fig. 8 is a diagram showing a modified example of the observation device of Fig. 1.
Fig. 9A is a diagram showing a modified example of the observation device of Fig. 1.
Fig. 9B is a diagram showing a modified example of the observation device of Fig. 1.
Fig. 10 is a diagram showing a modified example of the observation device of Fig. 1.
Fig. 11 is a diagram showing a configuration of an observation device of a second embodiment.
Fig. 12 is an enlarged view of a part of the observation device of Fig. 11.
Fig. 13 is a diagram showing a functional configuration of an analysis unit of Fig. 11.
Fig. 14 is a graph for describing how to determine a Rayleigh scattering coefficient and a reflection intensity in the analysis unit of Fig. 11.
Fig. 15 is a graph for describing a wavelength range suitable for observing a state of a cell population.
Fig. 16 is a diagram showing a modified example of the observation device of Fig. 11.
Fig. 17 is a diagram showing a modified example of the observation device of Fig. 11.
Fig. 18A is a diagram showing a modified example of the observation device of Fig. 11.
Fig. 18B is a diagram showing a modified example of the observation device shown in Fig. 11.
Fig. 19 is a graph for describing how to determine a Rayleigh scattering coefficient and a reflection intensity in the observation devices of Figs. 18A and 18B.
Fig. 20 is a diagram showing a modified example of the observation device shown in Fig. 11.
Fig. 21 is a diagram showing a modified example of the observation device shown in Fig. 11.

### Description of Embodiments

### Problems to be Solved by the Present Disclosure

For example, a plurality of cells form a planar population or a multilayered stacked population through cell proliferation, thereby forming the above-described cell population. In such a cell population, the number of cells and the cell density may vary depending on its position. Information such as the number of cells and the cell density for each position in the cell population is an important indicator for detailed evaluation of a state of the cell population. The intensity of light scattered and reflected from a cell population when the cell population is irradiated with light depends on the number of cells and the cell density within the cell population. Accordingly, by observing the intensity of emission light from the cell population, it is possible to obtain information including the number of cells and the cell density. When a cell population is irradiated with light, in addition to light scattering and reflection from the cell population, light absorption in the cell population and culture medium also occurs. For that reason, a mixture of a plurality of phenomena including light scattering and reflection, and light absorption is reflected in the intensity of emission light from a cell population.

In the method of using a transmission microscope described above, a mixture of a plurality of phenomena such as scattering, reflection, and light absorption is reflected in the obtained microscope image. It is difficult to distinguish between the influence of scattering and reflection by a cell population and the influence of light absorption by the cell population and the like, from such an observed image (that is, the emission light). Accordingly, it is difficult to obtain detailed information including the number of cells and the cell density using this method. The same can be said for the methods disclosed in Patent Literatures 1 and 2. In addition, the methods disclosed in Patent Literatures 1 and 2 make it possible to evaluate the quality of a cell population on a population-by-population basis using light intensity distribution, but it is difficult to obtain detailed information such as the number of cells and the cell density at a specific position within the cell population.

The methods disclosed in Patent Literatures 3 and 4 (methods using OCT) use light interference to scan a cell population three-dimensionally, which makes it time-consuming to observe a state of the cell population. These methods require observing the cell population when it has been taken out from a culture vessel in which environmental conditions such as temperature and atmospheric gas are controlled and maintained at conditions suitable for culturing the cell population. For that reason, when its invasive nature is considered, it is difficult to use OCT for quality control or 100% inspection of the cell population during culture.

The present disclosure provides an observation device and an observation method in which a state of a cell population can be observed in detail and efficiently.

### Effects of the Present Disclosure

According to the present disclosure, it is possible to provide an observation device and an observation method in which a state of a cell population can be observed in detail and efficiently.

### Description of Embodiments of Present Disclosure

First, embodiments of the present disclosure will be listed and described.
(1) An observation device of the present disclosure is an observation device configured to observe a state of a cell population configured of a plurality of cells. The observation device includes: at least one irradiation unit that is disposed at a position facing the cell population and irradiates at least one irradiation region set in the cell population with irradiation light having at least one wavelength included in the ranges of 650 nm to 850 nm and 1060 nm to 1090 nm; at least one light receiving unit that is disposed at a position facing the cell population and receives emission light emitted from the cell population as a result of irradiating the irradiation region with the irradiation light; and an analysis unit that is communicatively connected to the light receiving unit and determines a parameter including at least one of the number of cells and a cell density of the cell population in the irradiation region on the basis of an intensity of the emission light.

In general, when a cell population is irradiated with light, phenomena such as light scattering, reflection, and absorption may occur in and around the cell population. A mixture of these phenomena is reflected in an intensity of light emitted from the cell population in response to the irradiation of light. For this reason, intensities of scattering, reflection, and absorption can be observed by observing an amount of change in or a relative value of an emission light intensity relative to an irradiation light intensity (hereinafter, an "amount of change in or a relative value of an emission light intensity relative to an irradiation light intensity" will be referred to as an "amount of change in light intensity"). In the following, the terms "intensity of absorption" and "absorption intensity" indicate an "amount of change in light intensity due to absorption". The terms "intensity of scattering" and "scattering intensity" indicate an "amount of change in light intensity due to scattering". The terms "intensity of reflection" and "reflection intensity" indicate an "amount of change in light intensity due to reflection". Among these phenomena, the amounts of changes in light intensity due to scattering and reflection depend on parameters including at least one of the number of cells and the cell density in the cell population. The amount of change in light intensity due to absorption depends on chemical reactions in and around the cell population. Accordingly, in order to quantitatively determine the parameters such as the number of cells and the cell density, the amounts of changes in light intensity due solely to scattering and reflection may be acquired. After extensive investigation into methods for acquiring changes in light intensity due solely to scattering and reflection, the inventors have found that it is effective to irradiate a cell population with irradiation light having at least one wavelength included in the ranges of 650 nm to 850 nm and 1060 nm to 1090 nm. For example, in a culture medium in which a cell population may be disposed, the wavelength ranges of 650 nm to 850 nm and 1060 nm to 1090 nm are ranges in which light absorption by the culture medium is sufficiently smaller than light scattering and reflection by cells, and thus the influence of light absorption by the culture medium on an intensity of emission light can be ignored. In the cells, the wavelength ranges of 650 nm to 850 nm and 1060 nm to 1090 nm are also ranges in which light absorption by the cells is sufficiently smaller than scattering and reflection by the cells, and thus the influence of light absorption by the cells on the intensity of emission light can be ignored. Accordingly, in the case of using the irradiation light having at least one wavelength included in the ranges of 650 nm to 850 nm and 1060 nm to 1090 nm for irradiating a cell population, it is possible to quantitatively determine the parameters including at least one of the number of cells and the cell density as indicators of a state of the cell population on the basis of the amounts of changes in light intensity due solely to scattering and reflection. In the above observation device, the irradiation light is radiated to each irradiation region of the cell population, and thus the parameters including at least one of the number of cells and the cell density can be determined for each irradiation region on the basis of the intensity of the emission light from the irradiation region of the cell population. Thus, a state of a specific position in the cell population can be observed. Accordingly, according to the above observation device, it is possible to observe the state of the cell population in more detail. Unlike methods using OCT, according to the above observation device, it is possible to observe the state of the cell population efficiently in a short amount of time through a simple task of irradiating the cell population with the irradiation light.

(2) In the observation device described in the above (1), the irradiation unit may include a laser light source using a semiconductor optical element that emits light having one wavelength included in the ranges of 650 nm to 850 nm and 1060 nm to 1090 nm as the irradiation light. In this case, by using the semiconductor light emitting element with a stable light intensity as the light source, the risk of fluctuations in the intensity of the emission light due to factors other than the state of the cell population (for example, instability in the intensity of the irradiation light) can be reduced. Thus, the state of the cell population can be observed more reliably based on the intensity of the emission light.

(3) In the observation device described in the above (1) or (2), the light receiving unit may be disposed at a position opposite to the irradiation unit with the cell population interposed therebetween, and may receive part of the irradiation light emitted from the irradiation unit and transmitted through the cell population as the emission light. In this case, a position of the light receiving unit relative to the irradiation unit can be easily adjusted.

(4) In the observation device described in any of the above (1) to (3), the irradiation unit may include one or more light sources that emit the irradiation light, and may irradiate each of a plurality of the irradiation regions set in the cell population with the irradiation light. The light receiving unit may include one or more optical sensors on which the emission light is incident, and may receive a plurality of the irradiation lights transmitted through the cell population as a plurality of the emission lights. In this way, in the case of irradiating each irradiation region of the cell population with the irradiation light simultaneously, it is possible to observe the state of each irradiation region of the cell population more efficiently in a shorter amount of time than in the case of irradiating each irradiation region of the cell population sequentially.

(5) In the observation device described in any of the above (1) to (4), the irradiation unit may irradiate the irradiation region of the cell population with first irradiation light having a first wavelength included in the ranges of 650 nm to 850 nm and 1060 nm to 1090 nm, and second irradiation light having a second wavelength included in the ranges of 650 nm to 850 nm and 1060 nm to 1090 nm, which is different from the first wavelength. The light receiving unit may receive the first emission light and the second emission light, which have different wavelengths from each other, and which are emitted from the cell population as a result of radiating the first irradiation light and the second irradiation light. The analysis unit may determine each of the number of cells and the cell density in the irradiation region on the basis of an intensity of the first emission light and an intensity of the second emission light. Among the phenomena of light scattering, reflection, and absorption that occur in a cell population, light scattering and reflection can be broadly divided into Rayleigh scattering, which occurs due to internal molecular structures of cells forming the cell population, and reflection, which occurs due to density fluctuations within the cell population. The amount of change in light intensity due to Rayleigh scattering depends on the number of cells of the cell population. The amount of change in light intensity due to reflection depends on the cell density of the cell population. The amount of change in light intensity due to Rayleigh scattering is wavelength dependent. The amount of change in light intensity due to reflection is not wavelength dependent. For that reason, in the case of observing the state of the cell population using light of a plurality of wavelengths, by utilizing the difference in wavelength dependence, it is possible to separately determine the amount of change in light intensity due to the wavelength-dependent Rayleigh scattering and the amount of change in light intensity due to the wavelength-independent reflection. That is, it is possible to quantitatively determine the number of cells and the cell density separately. Thus, the state of the cell population can be observed in more detail.

(6) In the observation device described in the above (5), the irradiation unit may include a first light source that emits the first irradiation light, a second light source that emits the second irradiation light, and a switching unit that switches an optical path of the first irradiation light and an optical path of the second irradiation light so that the first irradiation light and the second irradiation light are selectively radiated to the irradiation region of the cell population. The light receiving unit may include an optical sensor that selectively receives the first emission light and the second emission light emitted from the cell population. In this case, the intensity of the first emission light and the intensity of the second emission light can be detected separately using one optical sensor. As a result, since there is no need to separately prepare an optical sensor for detecting the intensity of the first emission light and an optical sensor for detecting the intensity of the second emission light, complexity of the configuration of the light receiving unit can be avoided.

(7) In the observation device described in the above (5), the irradiation unit may include a first light source that emits the first irradiation light, a second light source that emits the second irradiation light, and a combining unit that combines the first irradiation light and the second irradiation light to generate combined irradiation light and irradiates the irradiation region of the cell population with the combined irradiation light. The light receiving unit may include a spectroscopic unit that spectrally separates the combined emission light emitted from the cell population as a result of radiating the combined irradiation light into each wavelength, a first optical sensor that detects the intensity of the first emission light spectrally separated from the combined emission light, and a second optical sensor that detects the intensity of the second emission light spectrally separated from the combined emission light. In this case, the intensity of the first emission light and the intensity of the second emission light can be detected simultaneously using the combined irradiation light and the combined emission light, and thus the state of the cell population can be observed more efficiently in a shorter amount of time based on the intensity of the first emission light and the intensity of the second emission light.

(8) In the observation device described in the above (5), the irradiation unit may include a light source that emits light including the first irradiation light and the second irradiation light, and a filter switching unit that includes a first filter which transmits only the first irradiation light and a second filter which transmits only the second irradiation light, and switches between a position of the first filter and a position of the second filter so that the first filter or the second filter is disposed on an optical path of the light between the light source and the cell population. The light receiving unit may include an optical sensor that selectively receives the first emission light and the second emission light emitted from the cell population. In this case, for example, by using an inexpensive lamp light source as the light source, the cost of the observation device can be reduced as compared to when an expensive laser light source is used as the light source.

(9) An observation method of the present disclosure is an observation method for observing a state of a cell population configured of a plurality of cells. The observation method includes the steps of: irradiating at least one irradiation region set in the cell population with irradiation light having at least one wavelength included in the ranges of 650 nm to 850 nm and 1060 nm to 1090 nm; receiving emission light emitted from the cell population as a result of irradiating the irradiation region with the irradiation light; and determining a parameter including at least one of the number of cells and a cell density of the cell population in the irradiation region on the basis of an intensity of the emission light. According to this observation method, the same effects as the observation device described above can be obtained.

(10) In the observation method described in the above (9), in the step of irradiating the irradiation region, first irradiation light having a first wavelength included in the ranges of 650 nm to 850 nm and 1060 nm to 1090 nm, and second irradiation light having a second wavelength included in the ranges of 650 nm to 850 nm and 1060 nm to 1090 nm, which is different from the first wavelength, may be radiated to the irradiation region. In the step of receiving the emission light, the first emission light and the second emission light, which have different wavelengths from each other, and which are emitted from the cell population as a result of radiating the first irradiation light and the second irradiation light, may be received. In the step of determining the parameter, the number of cells and the cell density in the irradiation region may be determined respectively on the basis of an intensity of the first emission light and an intensity of the second emission light. In this case, the same effects as in the above (5) can be obtained.

### Details of Embodiments of Present Disclosure

Specific examples of an observation device and an observation method of the present disclosure will be described below with reference to the drawings. The present disclosure is not limited to these examples but is indicated by the scope of the claims, and is intended to include meanings equivalent to the scope of the claims and all modifications within the scope.

### First Embodiment

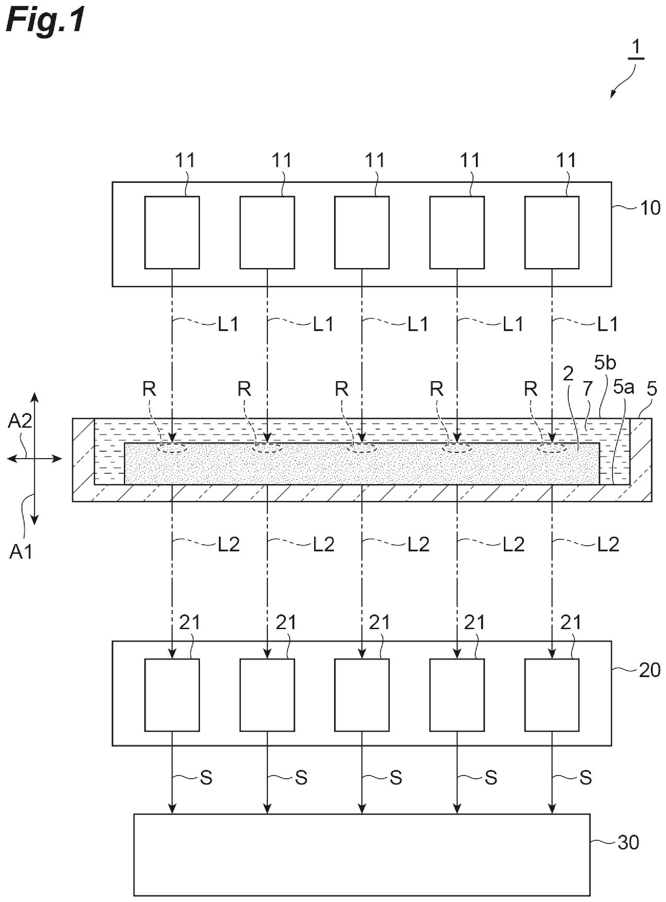

Fig. 1 is a diagram showing a configuration of an observation device 1 according to a first embodiment. The observation device 1 is a device for observing a state of a cell population 2. The cell population 2 is a cluster formed by a collection of a plurality of cells, that is, a cluster in which a plurality of cells are aggregated. The cell population 2 can also be described as a cell population in which a plurality of cells are collected in a single-layered planar shape or in a multilayered three-dimensional shape. The cell population 2 includes various cell forms, such as a collection of a plurality of cells called a colony, a collection arranged in a two-dimensional sheet shape, a two-dimensional cluster, and a three-dimensional cluster, tissue, or organ called a spheroid, an organoid, and an assembloid. Cells included in the cell population 2 include cells sampled from an animal or human, stem cells sampled from an animal or human, stem cells produced on the basis of cells sampled from an animal or human, or cells differentiated from stem cells. A single cell population 2 may contain a plurality of cell types.

The "state" of the cell population 2 observed by the observation device 1 is represented by a parameter indicating at least one of the "number of cells" and the "cell density" of the cell population 2. In the present embodiment, a parameter called a "degree of aggregation," which comprehensively represents the "number of cells" and the "cell density" of the cell population 2, is used as an indicator showing the "state" of the cell population 2. The "degree of aggregation" is defined by the "number of cells" × "cell density" (that is, a value obtained by the "number of cells" multiplied by the "cell density").

The observation device 1 irradiates each irradiation region R set in the cell population 2 with irradiation light L1, and determines the "degree of aggregation" of the cell population 2 for each irradiation region R on the basis of an intensity of emission light L2 from the cell population 2. As a result, information including the "number of cells" and the "cell density" is obtained for each irradiation region R. The "number of cells" in the irradiation region R indicates the total number of cells in the irradiation region R. The "cell density" in the irradiation region R indicates the number of cells present in a unit volume of the irradiation region R. The cell population 2 is formed, for example, by proliferation of a single cell such that a plurality of cells are stacked in multiple layers. In the cell population 2 formed in this way, the "number of cells" and the "cell density" differ for each position (region). Accordingly, obtaining information on the "number of cells" and the "cell density" for each irradiation region R is important for evaluating the state of the cell population 2 in detail.

As shown in Fig. 1, the observation device 1 includes, for example, an irradiation module 10, a light receiving module 20, and an analysis unit 30. The irradiation module 10 is disposed at a position facing the cell population 2. The light receiving module 20 is disposed, for example, at a position opposite to the irradiation module 10 with the cell population 2 interposed therebetween. The cell population 2 is housed in a container 5, for example, together with a culture medium 7. The container 5 is cylindrical with an opening 5b at one end. The observation device 1 may include a placement table on which the container 5 is placed.

The cell population 2 is disposed on a bottom surface 5a of the container 5 while it is immersed in the culture medium 7. The cell population 2 may be in contact with the bottom surface 5a or may be separated from the bottom surface 5a. The container 5 is made of, for example, a glass or resin material that is transmissive to the emission light L2 emitted from the cell population 2. In the following, in a vertical direction A1 along a normal to the bottom surface 5a, a direction from the bottom surface 5a to the opening 5b may be referred to as "upward," and a direction from the opening 5b to the bottom surface 5a may be referred to as "downward." A direction perpendicular to the vertical direction A1 is a lateral direction A2. The transmissive material is, for example, a material that has a transmittance of 20% or more for light having wavelengths in the ranges of 650 nm to 850 nm and 1060 nm to 1090 nm when the material is 1 mm thick.

The irradiation module 10 includes, for example, a plurality of irradiation units 11 arranged two-dimensionally when viewed from above. For example, the plurality of irradiation units 11 are arranged in a planar shape in the lateral direction A2 perpendicular to the vertical direction A1 and a longitudinal direction (depth direction relative to the paper surface in Fig. 1) perpendicular to both the vertical direction A1 and the lateral direction A2. A plurality of irradiation regions R are set in the cell population 2. The plurality of irradiation regions R are a plurality of regions set by dividing the cell population 2 when the cell population 2 is viewed from above, and are regions to be irradiated with the irradiation light L1. The plurality of irradiation regions R are arranged two-dimensionally when viewed from above. For example, the plurality of irradiation regions R are arranged in a planar shape in the lateral direction A2 and the longitudinal direction (depth direction relative to the paper surface in Fig. 1). The plurality of irradiation units 11 are respectively disposed above the plurality of irradiation regions R. That is, the plurality of irradiation units 11 are disposed at positions respectively facing the plurality of irradiation regions R in a one-to-one relationship in the vertical direction A1. The plurality of irradiation units 11 respectively irradiate the plurality of irradiation regions R with the irradiation light L1. For example, the respective irradiation units 11 have the same configuration as each other. The respective irradiation units 11 may have different configurations from each other.

The light receiving module 20 includes, for example, a plurality of light receiving units 21 arranged two-dimensionally when viewed from above. For example, the plurality of light receiving units 21 are arranged in a planar shape in the lateral direction A2 and the longitudinal direction (depth direction relative to the paper surface in Fig. 1). The plurality of light receiving units 21 are disposed below the plurality of irradiation regions R. The plurality of light receiving units 21 are disposed at positions respectively facing the plurality of irradiation units 11 in a one-to-one relationship in the vertical direction A1 with the cell population 2 interposed therebetween. Each of the light receiving units 21 receives the emission light L2 emitted from the cell population 2 as a result of irradiating the irradiation region R of the cell population 2 with the irradiation light L1. In the present embodiment, the emission light L2 is transmitted light of the irradiation light L1 transmitted through the cell population 2. Each of the light receiving units 21 outputs an electric signal S corresponding to the intensity of the emission light L2 to the analysis unit 30. For example, the respective light receiving units 21 have the same configuration as each other. The respective light receiving units 21 may have different configurations from each other.

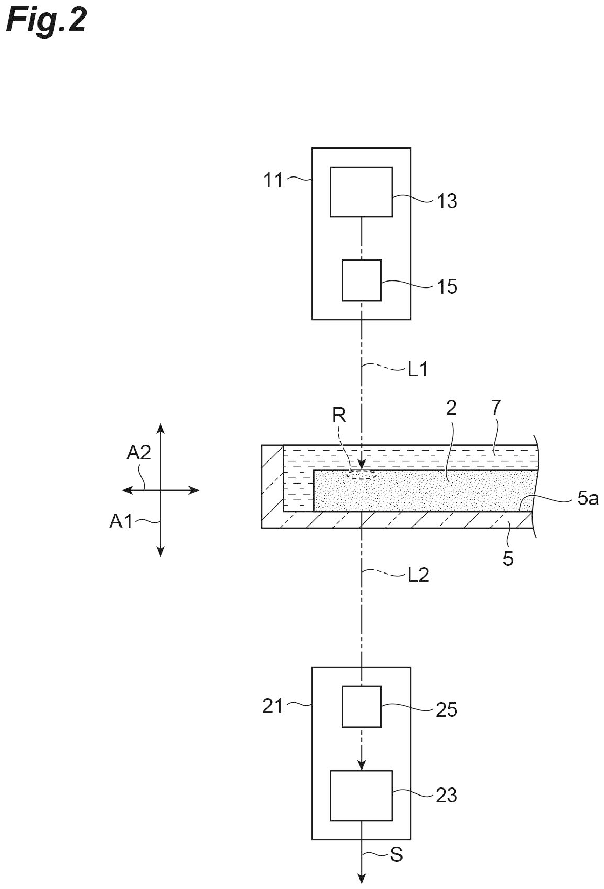

Fig. 2 is an enlarged view showing a part of the observation device 1. As shown in Fig. 2, the irradiation unit 11 includes, for example, a light source 13 and a lens 15. The light source 13 is disposed above the irradiation region R of the cell population 2. That is, the light source 13 is disposed at a position facing the irradiation region R of the cell population 2 in the vertical direction A1. The light source 13 emits the irradiation light L1 toward the irradiation region R. An optical axis of the irradiation light L1 emitted from the light source 13 is directed in the vertical direction A1. In one example, the optical axis of the irradiation light L1 is perpendicular to the bottom surface 5a and parallel to the vertical direction A1. The light source 13 is, for example, a laser light source using a laser diode (LD). The irradiation light L1 has one wavelength included in the ranges of 650 nm to 850 nm and 1060 nm to 1090 nm. The ranges of 650 nm to 850 nm and 1060 nm to 1090 nm indicate ranges including both the range of 650 nm to 850 nm and the range of 1060 nm to 1090 nm. Accordingly, it can be said that the irradiation light L1 has one wavelength included in at least one of the range of 650 nm to 850 nm and the range of 1060 nm to 1090 nm.

The light source 13 is not limited to a laser light source using a laser diode. For example, the light source 13 may be a light source using a semiconductor light emitting element such as a laser diode, a light emitting diode (LED), a super luminescent diode (SLD), a laser light source using an element other than a semiconductor light emitting element, or a lamp light source. For example, if a broadband light source such as a lamp light source is used, the irradiation light L1 may have, in addition to the wavelength included in the ranges of 650 nm to 850 nm and 1060 nm to 1090 nm, one or more wavelengths different from that wavelength. In this case, the one or more wavelengths different from that wavelength may be wavelengths included in the ranges of 650 nm to 850 nm and 1060 nm to 1090 nm, or wavelengths outside the ranges of 650 nm to 850 nm and 1060 nm to 1090 nm.

The lens 15 is disposed on an optical path of the irradiation light L1 between the light source 13 and the cell population 2. The lens 15 is, for example, a condensing lens that condenses the irradiation light L1 from the light source 13. The lens 15 may be a collimating lens that collimates the irradiation light L1. The irradiation light L1 emitted from the light source 13 passes through the lens 15 and is irradiated onto the irradiation region R of the cell population 2. Part of the irradiation light L1 is transmitted through the cell population 2 and is emitted as the emission light L2.

The light receiving unit 21 includes, for example, an optical sensor 23 and a lens 25. The optical sensor 23 is disposed below the irradiation region R of the cell population 2. That is, the optical sensor 23 is disposed at a position opposite to the light source 13 in the vertical direction with the irradiation region R of the cell population 2 interposed therebetween. Accordingly, the light receiving unit 21 faces the light source 13 in the vertical direction A1 with the cell population 2 interposed therebetween. The optical sensor 23 is disposed on an optical path of the emission light L2 emitted from the cell population 2, that is, on an extension line of the optical axis of the irradiation light L1. The optical axis of the emission light L2 is, for example, perpendicular to the bottom surface 5a on which the cell population 2 is placed and parallel to the vertical direction A1. The size of a field of view of the optical sensor 23 is suitably, for example, the same as or smaller than a size of the cells forming the cell population 2. For example, the diameter of the field of view of the optical sensor 23 may be 20 µm or less.

The lens 25 is disposed on the optical path of the emission light L2 between the cell population 2 and the optical sensor 23. The lens 25 is, for example, an objective lens. The lens 25 may be a collimating lens. The emission light L2 emitted from the cell population 2 passes through the lens 25 and is incident on the optical sensor 23. The optical sensor 23 receives the emission light L2 and outputs an electric signal S corresponding to the intensity of the emission light L2. If the irradiation light L1 contains a plurality of wavelengths, a filter that transmits light of a specific wavelength may be disposed at any position between the light source 13 and the optical sensor 23.

As shown in Fig. 1, the analysis unit 30 is communicatively connected to the plurality of light receiving units 21 of the light receiving module 20. The analysis unit 30 may be communicatively connected to the plurality of irradiation units 11 of the irradiation module 10. The analysis unit 30 receives the electric signal S from each optical sensor 23. On the basis of the received electric signal S, the analysis unit 30 determines the "degree of aggregation," which represents the "number of cells" and the "cell density" of the cell population 2.

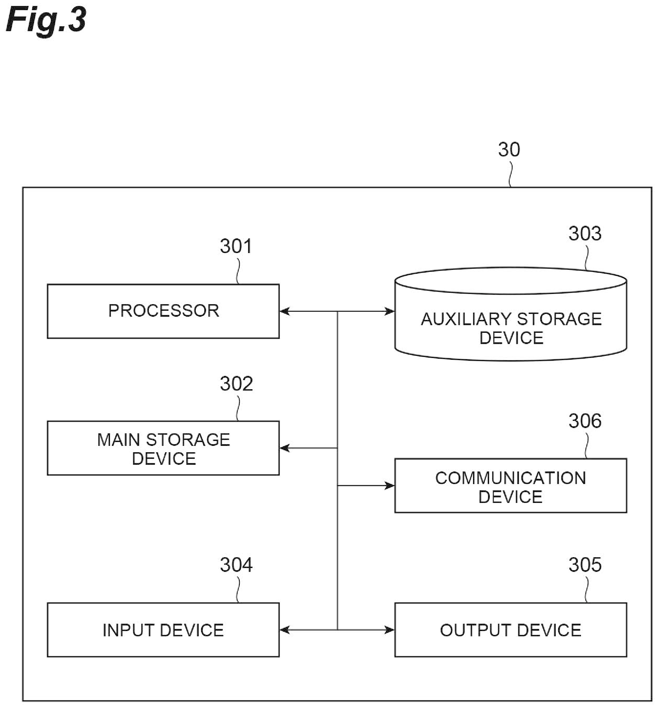

Fig. 3 is a diagram showing a hardware configuration of the analysis unit 30. The analysis unit 30 is physically one or more computers. As shown in Fig. 3, the analysis unit 30 physically includes hardware such as one or more processors 301, a main storage device 302, an auxiliary storage device 303, an input device 304, an output device 305, and a communication device 306. The analysis unit 30 is configured of one or more physical computers configured of this physical hardware and software such as programs.

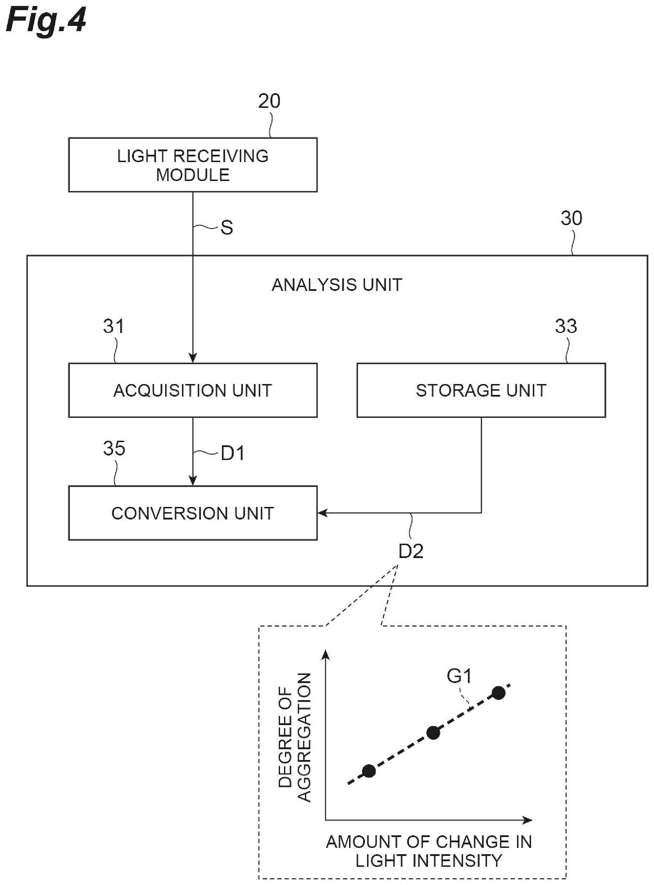

Fig. 4 is a diagram showing a functional configuration of the analysis unit 30. For example, the analysis unit 30 includes, as functional components, an acquisition unit 31, a storage unit 33, and a conversion unit 35. Each functional component of the analysis unit 30 is implemented by executing a program on the hardware of the physical computer described above.

The acquisition unit 31 acquires the intensity of the emission light L2 indicated by the electric signal S from the light receiving module 20 for each irradiation region R of the cell population 2. The acquisition unit 31 provides the conversion unit 35 with light intensity information D1 indicating the intensity of the emission light L2 for each irradiation region R of the cell population 2.

The storage unit 33 stores conversion information D2 for determining the "degree of aggregation" of the cell population 2 on the basis of the intensity of the emission light L2. The conversion information D2 includes, for example, a calibration curve G1 that shows a relationship between an amount of change in light intensity obtained from the light intensity information D1 and the "degree of aggregation" of the cell population 2. The calibration curve G1 is a graph plotting the relationship between the amount of change in light intensity and the "degree of aggregation" of the cell population 2. The amount of change in light intensity is a relative ratio of the intensity of the emission light L2 to the intensity of the irradiation light L1, and is a standardized value of the intensity of the emission light L2. The amount of change in light intensity is defined based on a value obtained by dividing the intensity of the emission light L2 by the intensity of the irradiation light L1 (-10Log(intensity of emission light L2/intensity of irradiation light L1)).

As shown in the calibration curve G1 in Fig. 4, the amount of change in light intensity decreases as the "degree of aggregation" of the cell population 2 decreases, and increases as the "degree of aggregation" increases. For that reason, if a magnitude of the amount of change in light intensity is known, the "degree of aggregation" can be estimated. By preparing a sample of the cell population 2 whose "degree of aggregation" is already known (or a sample having a "degree of aggregation" serving as a reference) and irradiating the sample with irradiation light L1 using the observation device 1, the calibration curve G1 showing a relationship between the amount of change in light intensity and the "degree of aggregation" of the sample can be created in advance.

The storage unit 33 stores the calibration curve G1 created in advance in this way and provides the conversion information D2 including the calibration curve G1 to the conversion unit 35. The conversion information D2 may include, instead of or together with the calibration curve G1, a conversion formula (conversion equation) showing the relationship between the amount of change in light intensity and the "degree of aggregation" of the cell population 2. The conversion information D2 may include, in addition to the calibration curve G1, information required for converting the amount of change in light intensity to the "degree of aggregation" of the cell population 2. For example, the conversion information D2 may include the intensity of the irradiation light L1 required for deriving the amount of change in light intensity.

The conversion unit 35 uses the light intensity information D1 and the conversion information D2 to determine the "degree of aggregation" of the cell population 2 for each irradiation region R. First, the conversion unit 35 calculates the amount of change in light intensity, which is the relative ratio between the intensity of the emission light L2 and the intensity of the irradiation light L1. Next, the conversion unit 35 converts the calculated amount of change in light intensity into the "degree of aggregation" of the cell population 2 by referring to the calibration curve G1. As described above, the "degree of aggregation" of the cell population 2 is a parameter that includes the "number of cells" and the "cell density" of the cell population 2, and is an indicator of the state of the cell population 2. Accordingly, by determining the "degree of aggregation" for each irradiation region R of the cell population 2, it is possible to quantitatively evaluate the state of each irradiation region R of the cell population 2. The analysis unit 30 may output the observation results, including the "degree of aggregation" of the cell population 2 determined by the conversion unit 35, using an output device 305 such as a monitor or printer, for example.

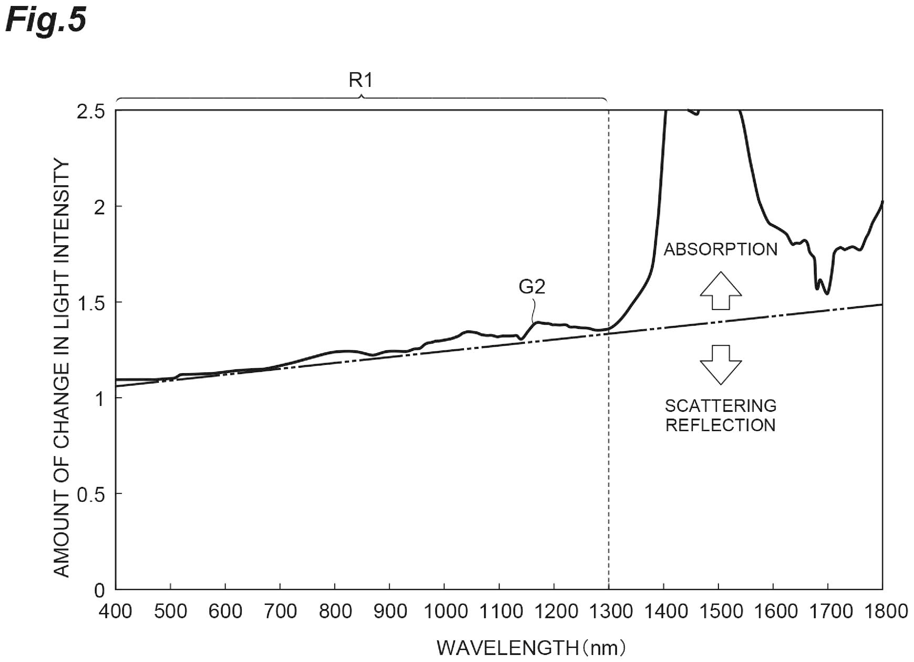

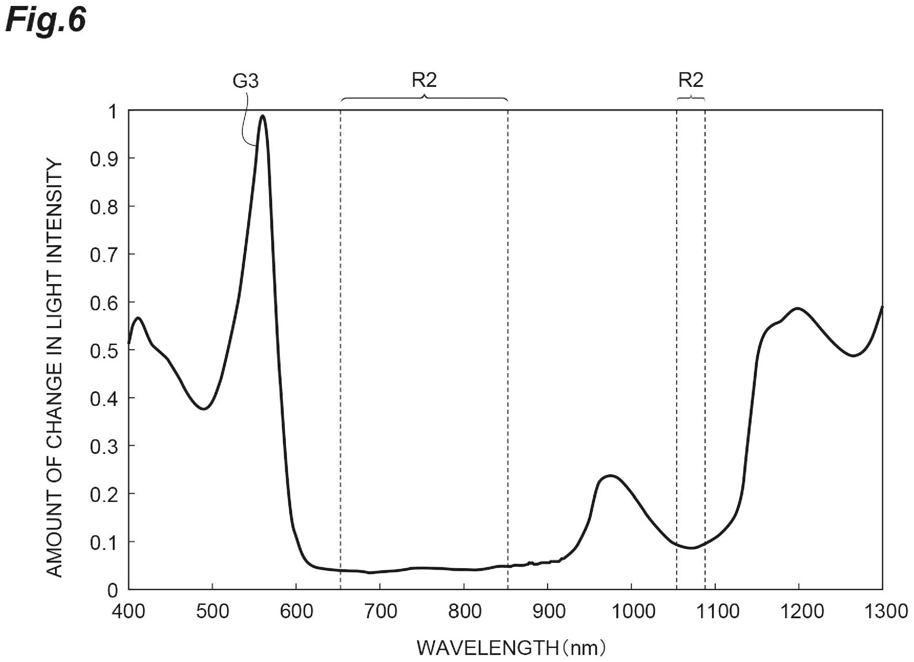

With reference to Figs. 5 and 6, the reasons for using the irradiation light L1 in the wavelength ranges of 650 nm to 850 nm and 1060 nm to 1090 nm for irradiating the cell population 2 will be described below.

Fig. 5 is a graph showing a spectrum of the amount of change in light intensity of the cell population 2. In Fig. 5, the vertical axis represents the amount of change in light intensity of the cell population 2 and the horizontal axis represents the wavelength [nm]. The vertical axis in Fig. 5 can also be said to represent a scattering intensity, a reflection intensity, and an absorption intensity of the cell population 2. Fig. 6 is a graph showing a spectrum of the amount of change in light intensity of the culture medium 7. In Fig. 6, the vertical axis represents the amount of change in light intensity of the culture medium 7, and the horizontal axis represents the wavelength [nm]. Since no scattering and reflection occur in the culture medium 7, the vertical axis in Fig. 6 can also be said to represent an absorption intensity of the culture medium 7.

When light is irradiated onto the cell population 2 in the culture medium 7, light absorption occurs in the cell population 2 and the culture medium 7, resulting in light scattering and reflection from the cell population 2. A mixture of a plurality of phenomena including light scattering and reflection, and light absorption is reflected in the intensity of light emitted (transmitted or reflected) from the cell population 2 as a result of irradiating the cell population 2 with light. Among these phenomena, the intensity of light due to scattering and reflection depends on parameters such as the "number of cells" and the "cell density" of the cell population 2. Accordingly, if the intensity of light due solely to scattering and reflection can be acquired, it is possible to quantitatively determine the "degree of aggregation" of the cell population 2, which is a parameter that includes the "number of cells" and the "cell density."

As shown in graph G2 in Fig. 5, there are many peaks in the wavelength range of 1300 nm or higher in which the amount of change in light intensity of the cell population 2 changes sharply. These peaks are caused by light absorption in the cell population 2. Accordingly, in the wavelength range in which there are many peaks, the influence of light absorption on the intensity of light emitted from the cell population 2 is significant, and thus it is difficult to obtain the amount of change in light intensity due solely to scattering and reflection. It can be seen that, in the wavelength range R1 of 1300 nm or less, the amount of change in light intensity changes extremely gradually, without any peaks. In this wavelength range R1, the influence of light absorption on the intensity of light emitted from the cell population 2 is sufficiently small and negligible as compared to the influence of scattering and reflection, and thus the amount of change in light intensity due solely to scattering and reflection can be easily obtained.

As shown in graph G3 in Fig. 6, within the wavelength range R1 of 1300 nm or less, in the wavelength ranges R2 of 650 nm to 850 nm and 1060 nm to 1090 nm, it can be seen that the amount of change in light intensity due solely to light absorption by the culture medium 7 is sufficiently small as compared to the amount of change in light intensity due to the light scattering and reflection by the cells as shown in Fig. 5. Accordingly, if light in the wavelength ranges of 650 nm to 850 nm and 1060 nm to 1090 nm is irradiated onto the cell population 2 in the culture medium 7, the influence of light absorption by the culture medium 7 can be ignored. That is, it is possible to prevent the intensity of light emitted from the cell population 2 from reflecting light absorption by the culture medium 7. The culture medium 7 may contain water, amino acids, vitamins, salts, and glucose. For example, an indicator such as phenol red may be added to the culture medium 7. If the influence of light absorption by the culture medium 7 can be ignored, it is possible to obtain the amount of change in light intensity that reflects only scattering and reflection by the cell population 2. Ultraviolet light having a wavelength shorter than 400 nm is not appropriate for irradiating the cell population 2 because it may cause damage to the cell population 2.

Accordingly, in the case of using the irradiation light L1 having wavelengths included in the wavelength ranges R2 of 650 nm to 850 nm and 1060 nm to 1090 nm for irradiating the cell population 2, as described above, the influence of absorption of the irradiation light L1 by the cell population 2 and the culture medium 7 can be ignored, and thus it is possible to obtain the intensity of the emission light L2 that reflects only scattering and reflection of the irradiation light L1 by the cell population 2. As a result, it is possible to quantitatively determine the "degree of aggregation" of the cell population 2 on the basis of the intensity of the emission light L2 obtained. Thus, in the present embodiment, on the basis of the spectrum of the amount of change in light intensity of the cells and the spectrum of the amount of change in light intensity of the culture medium 7, as the wavelength ranges in which the influence of absorption of the irradiation light L1 by the cell population 2 and the culture medium 7 can be ignored, the wavelength ranges R2 of 650 nm to 850 nm and 1060 nm to 1090 nm are selected.

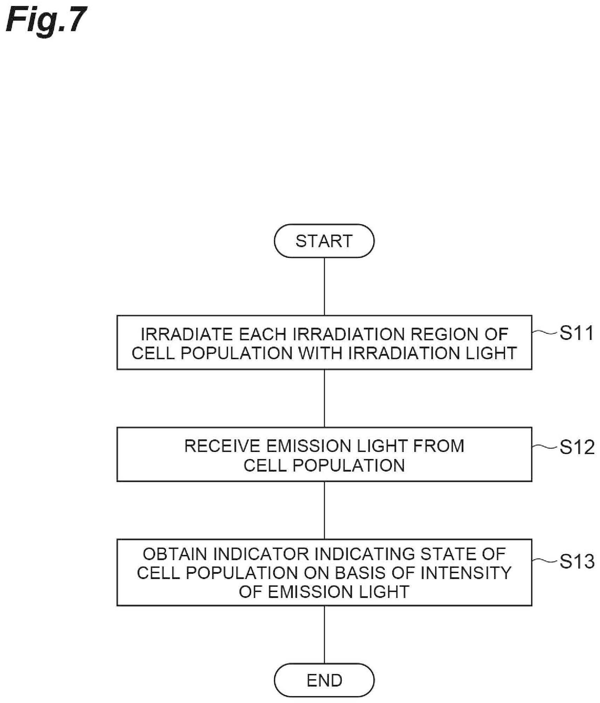

Steps of an observation method performed using the above-described observation device 1 will be described with reference to Fig. 7. Fig. 7 is a flowchart showing each step of the observation method.

First, each irradiation unit 11 arranged two-dimensionally above the cell population 2 irradiates each irradiation region R of the cell population 2 with the irradiation light L1 in the wavelength ranges of 650 nm to 850 nm and 1060 nm to 1090 nm (step S11). Part of the irradiation light L1 radiated to each irradiation region R of the cell population 2 is transmitted through the cell population 2 and is emitted from the cell population 2 as the emission light L2.

Next, each of the light receiving units 21 arranged two-dimensionally below the cell population 2 receives each emission light L2 emitted from the cell population 2 (step S12). Each of the light receiving units 21 outputs anelectric signal S, which corresponds to the intensity of each emission light L2 received, to the analysis unit 30.

Next, on the basis of the intensity of the emission light L2 indicated by each electric signal S, the analysis unit 30 determines the "degree of aggregation," which is a parameter that includes the "number of cells" and the "cell density" of the cell population 2, as an indicator of the state of the cell population 2 (step S13). Specifically, first, the conversion unit 35 of the analysis unit 30 calculates the amount of change in light intensity by dividing the intensity of the emission light L2 by the intensity of the irradiation light L1. Then, the conversion unit 35 converts the calculated amount of change in light intensity into the "degree of aggregation" of the cell population 2 by referring to the calibration curve G1 shown in Fig. 4. Using the "degree of aggregation" determined in this way, it is possible to quantitatively observe the state of each irradiation region R of the cell population 2.

The effects obtained by the first embodiment described above will be described. As described above, in the spectrum of the amount of change in light intensity of the culture medium 7, the wavelength ranges R2 of 650 nm to 850 nm and 1060 nm to 1090 nm (see Fig. 6) are ranges in which light absorption by the culture medium 7 in which the cell population 2 is disposed is sufficiently small as compared to the light scattering and reflection by the cells shown in Fig. 5. For that reason, in the case of using the irradiation light L1 having wavelengths in the wavelength ranges R2 of 650 nm to 850 nm and 1060 nm to 1090 nm for irradiating the cell population 2, the influence of light absorption by the culture medium 7 on the intensity of the emission light L2 can be ignored. In the spectrum of the amount of change in light intensity of the cell population 2, the wavelength ranges R2 of 650 nm to 850 nm and 1060 nm to 1090 nm are included in the wavelength range R1 (see Fig. 5) in which there are no sharp peaks generated due to light absorption by the cells, and thus the influence of light absorption by the cell population 2 on the intensity of the emission light L2 is sufficiently smaller than scattering and reflection occurring in the cell population 2, and can be ignored.

Accordingly, in the case of using the irradiation light L1 having at least one wavelength included in the wavelength ranges R2 of 650 nm to 850 nm and 1060 nm to 1090 nm for irradiating the cell population 2, the intensity of the emission light L2 due solely to scattering and reflection can be acquired, and thus, on the basis of the intensity of the emission light L2, it is possible to quantitatively determine the "degree of aggregation," which is a parameter that includes the "number of cells" and the "cell density," as an indicator of the state of the cell population 2. In the present embodiment, since the irradiation light L1 is radiated onto each irradiation region R of the cell population 2, the intensity of the emission light L2 can be obtained distinctively for each irradiation region R. That is, the "degree of aggregation" can be quantitatively determined for each irradiation region R of the cell population 2. Thus, since information including the "number of cells" and the "cell density" can be obtained at a specific position in the cell population 2, it is possible to observe the state of the cell population 2 in detail.

According to the present embodiment, unlike methods using OCT, the state of the cell population 2 can be observed efficiently in a short amount of time through a simple task of irradiating the cell population 2 with the irradiation light L1. As a result, it is possible to perform a total inspection to observe all the cell populations 2 to be observed, and to avoid complication of process management for observing the cell populations 2.

As in the present embodiment, the irradiation unit 11 may include the light source 13 using a laser diode that emits light having one wavelength included in the ranges of 650 nm to 850 nm and 1060 nm to 1090 nm as the irradiation light L1. The light source 13 may be a light source using a semiconductor light emitting element such as a laser diode, a light emitting diode diode, and a super luminescent diode. In this case, by using light from a light source using a semiconductor light emitting element with stable light intensity as the irradiation light L1, the risk of fluctuations in the intensity of the emission light L2 due to factors other than the state of the cell population 2 (for example, instability in the intensity of the irradiation light) can be reduced. Thus, the state of the cell population 2 can be observed more reliably based on the intensity of the emission light L2.

As in the present embodiment, the light receiving unit 21 may be disposed at a position opposite to the irradiation unit 11 with the cell population 2 interposed therebetween, and may receive part of the irradiation light L1 emitted from the irradiation unit 11 and transmitted through the cell population 2 as the emission light L2. In this case, the position of the light receiving unit 21 relative to the irradiation unit 11 can be easily adjusted.

As in the present embodiment, the plurality of irradiation units 11 may irradiate each of the plurality of irradiation regions R with the irradiation light L1. The plurality of light receiving units 21 may receive part of the irradiation light L1 emitted respectively from the plurality of irradiation units 11 and transmitted through the cell population 2 as a plurality of emission lights L2. In this way, in the case of irradiating each irradiation region R of the cell population 2 with the irradiation light L1 simultaneously, it is possible to observe the state of each irradiation region R of the cell population 2 more efficiently in a shorter amount of time than in the case of irradiating each irradiation region R of the cell population 2 with the irradiation light L1 sequentially.

The observation device and the observation method of the present disclosure are not limited to the first embodiment described above, and various other modifications are possible.

### <Modified Example 1>

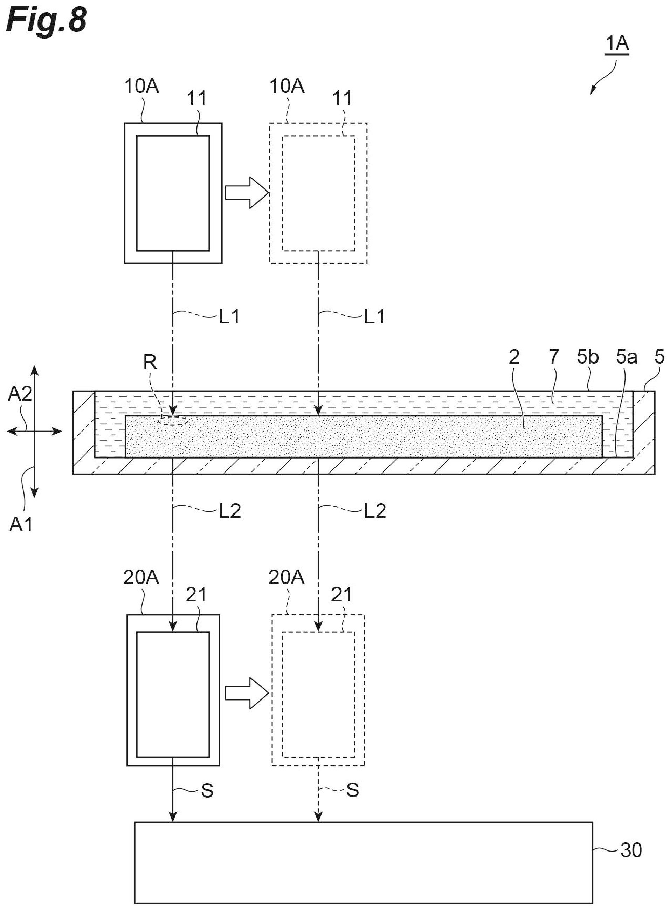

Fig. 8 is a diagram showing a modified example of the observation device 1. As in an observation device 1A shown in Fig. 8, an irradiation module 10A may include only one irradiation unit 11, and a light receiving module 20A may include only one light receiving unit 21. In this case, the irradiation unit 11 is disposed above one of the irradiation regions R of the cell population 2 and irradiates the irradiation region R with the irradiation light L1. The light receiving unit 21 is disposed at a position opposite to the irradiation unit 11 with the cell population 2 interposed therebetween. That is, the light receiving unit 21 is disposed below the irradiation region R irradiated with the irradiation light L1, and faces the irradiation unit 11 in the vertical direction A1 with the cell population 2 interposed therebetween.

The irradiation unit 11 and the light receiving unit 21 are configured to be movable in the lateral direction A2 perpendicular to the vertical direction A1, and the longitudinal direction (depth direction relative to the paper surface in Fig. 1) perpendicular to both the vertical direction A1 and the lateral direction A2. The irradiation unit 11 sequentially irradiates each irradiation region R of the cell population 2 with the irradiation light L1 while moving in the lateral direction A2 and the longitudinal direction. Similarly, the light receiving unit 21 also sequentially receives each emission light L2 emitted from the cell population 2 as a result of irradiating each irradiation region R with the irradiation light L1 while moving in the lateral direction A2 and the longitudinal direction in accordance with the movement of the irradiation unit 11.

The irradiation unit 11 and the light receiving unit 21 may be configured to be movable relative to the container 5 in which the cell population 2 is housed. Accordingly, the irradiation unit 11 and the light receiving unit 21 may be configured to move in the lateral direction A2 and the longitudinal direction while a position of the container 5 remains fixed. Conversely, the container 5 may be configured to move in the lateral direction A2 and the longitudinal direction while the respective positions of the irradiation unit 11 and the light receiving unit 21 remain fixed.

Since the observation device 1A shown in Fig. 8 can also quantitatively determine the "degree of aggregation," which is the parameter that includes the "number of cells" and the "cell density," for each irradiation region R of the cell population 2 on the basis of the intensity of each emission light L2 emitted from the cell population 2, the same effects as those of the first embodiment can be obtained. Since the observation device 1A can set each irradiation region R more finely for the cell population 2 in accordance with a movement width of the irradiation unit 11 in the lateral direction A2, it is possible to determine the "degree of aggregation" in a larger number of irradiation regions R of the cell population 2. That is, it is possible to observe the state of the cell population 2 in more detail.

### <Modified Example 2>

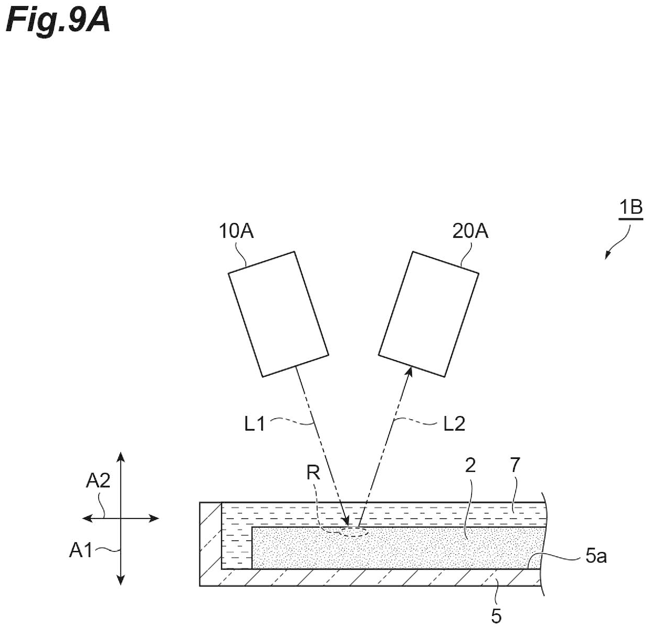

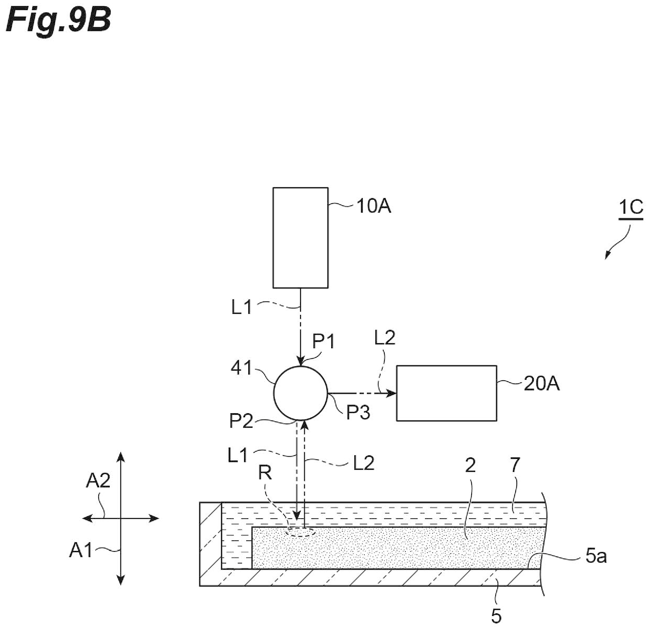

Figs. 9A and 9B are diagrams showing a modified examples of the observation device 1. Each of an observation device 1B shown in Fig. 9A and an observation device 1C shown in Fig. 9B includes, for example, the irradiation module 10A and the light receiving module 20A shown in Fig. 8 described above. Each of the observation devices 1B and 1C may include the irradiation module 10 and the light receiving module 20 of the first embodiment. In the observation devices 1B and 1C, the light receiving module 20A is disposed above the cell population 2, similarly to the irradiation module 10A. That is, of two regions sandwiching the cell population 2, the light receiving module 20A is disposed in the same region as the region in which the irradiation module 10A is located.

In the observation device 1B shown in Fig. 9A, the optical axis of the irradiation light L1 emitted from the irradiation module 10A is inclined with respect to the vertical direction A1. Part of the irradiation light L1 radiated to the irradiation region R of the cell population 2 is reflected on a surface of or inside the cell population 2. The reflected light reflected in the cell population 2 is emitted from the cell population 2 as the emission light L2. The optical axis of the emission light L2 is inclined with respect to the vertical direction A1.

The light receiving module 20A is disposed on the optical path of the emission light L2. The light receiving module 20A is disposed, for example, adjacent to the irradiation module 10A in the lateral direction A2. The light receiving module 20A receives the emission light L2 and detects the intensity of the emission light L2. Since the observation device 1B can also quantitatively determine the "degree of aggregation," which is the parameter including the "number of cells" and the "cell density," for each irradiation region R of the cell population 2 on the basis of the intensity of each emission light L2 from the cell population 2, the same effects as the first embodiment can be obtained.

In the observation device 1C shown in Fig. 9B, a circulator 41 is disposed above the irradiation region R of the cell population 2. The irradiation module 10A is disposed further above the circulator 41. The light receiving module 20A is disposed at a position away from the circulator 41 in the lateral direction A2. The circulator 41 is disposed at an intersection between the optical axis of the irradiation light L1 emitted from the irradiation module 10A and the optical axis of the emission light L2 incident on the light receiving module 20A. The circulator 41 may be optically coupled to the irradiation module 10A and the light receiving module 20A using lenses and optical fibers. A collimating lens or objective lens may be disposed between the circulator 41 and the cell population 2.

The circulator 41 is an optical component for separating two beams of light traveling in opposite directions from each other, and has three ports P1, P2, and P3 for the light. The irradiation light L1 emitted from the irradiation module 10A is incident on the port P1 of the circulator 41 and is emitted from the port P2 to the irradiation region R of the cell population 2. The emission light L2 emitted from the cell population 2 toward the port P2 is emitted from the port P3 to the light receiving module 20A. Since the observation device 1C can also quantitatively determine the "degree of aggregation," which is the parameter including the "number of cells" and the "cell density," for each irradiation region R of the cell population 2 on the basis of the intensity of each emission light L2 from the cell population 2, the same effects as the first embodiment can be obtained.

### <Modified Example 3>

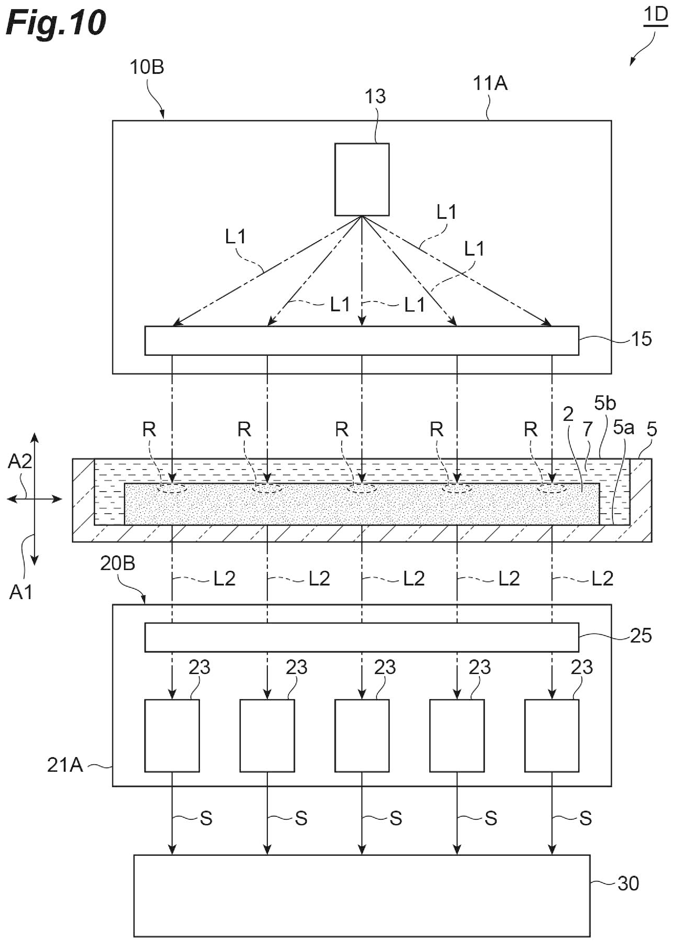

Fig. 10 is a diagram showing a modified example of the observation device 1. As in an observation device 1D shown in Fig. 10, an irradiation module 10B may include only one irradiation unit 11A, and a light receiving module 20B may include only one light receiving unit 21A. In the example shown in Fig. 10, the irradiation unit 11A includes one light source 13 and one lens 15. The light source 13 is configured to be able to uniformly irradiate a wide area with the irradiation light L1. The light source 13 is disposed above the cell population 2 and uniformly radiates the irradiation light L1 over a wide area that extends two-dimensionally when viewed from above. A range of the irradiation light L1 radiated from the light source 13 may be set in a region that extends in a planar shape in the lateral direction A2 and the longitudinal direction (depth direction relative to the paper surface in Fig. 10). The light source 13 radiates the irradiation light L1 simultaneously and uniformly to a plurality of irradiation regions R arranged in the lateral direction A2 and the longitudinal direction, which are set in the cell population 2.

The lens 15 is disposed on the optical path of the irradiation light L1 between the light source 13 and the cell population 2. The lens 15 is, for example, a collimating lens that collimates the irradiation light L1 emitted over a wide area from the light source 13. The lens 15 extends in a planar shape in the lateral direction A2 and the longitudinal direction so that it faces the light source 13 in the vertical direction A1 and all irradiation regions R set in the cell population 2 in the vertical direction A1. The irradiation light L1 emitted from the light source 13 in a diverging manner is collimated by the lens 15 and radiated onto each irradiation region R of the cell population 2. Then, part of each of a plurality of irradiation lights L1 is transmitted through the cell population 2 and is emitted as the plurality of emission lights L2.

The light receiving unit 21A includes a plurality of optical sensors 23 and one lens 25. The plurality of optical sensors 23 are respectively disposed below the plurality of irradiation regions R of the cell population 2. The light receiving unit 21A receives the emission light L2 emitted from the cell population 2 as a result of irradiating the irradiation region R of the cell population 2 with the irradiation light L1.

The lens 25 is disposed on the optical path of the plurality of emission lights L2 between the cell population 2 and the plurality of optical sensors 23. The lens 25 is, for example, an objective lens. The lens 25 may be a collimating lens. The lens 25 extends in a planar shape in the lateral direction A2 and the longitudinal direction so that it faces all irradiation regions R set in the cell population 2 in the vertical direction A1 and all optical sensors 23 in the vertical direction A1. The lens 25 faces the lens 15 in the vertical direction A1 with the cell population 2 interposed therebetween. Each emission light L2 emitted from the cell population 2 passes through the lens 25 and is incident on each optical sensor 23. Each optical sensor 23 receives the emission light L2 and outputs the electric signal S corresponding to the intensity of the emission light L2.

Since the observation device 1D shown in Fig. 10 can also quantitatively determine the "degree of aggregation," which is the parameter including the "number of cells" and the "cell density," for each irradiation region R of the cell population 2 on the basis of the intensity of each emission light L2 from the cell population 2, the same effects as the first embodiment can be obtained. The observation device 1D can irradiate each irradiation region R with the irradiation light L1 using one light source 13, and thus as compared to the case in which a plurality of light sources are used to irradiate the respective irradiation regions R with the irradiation light L1, the number of light sources can be reduced, and the device configuration can be simplified.

In the observation device 1D, the irradiation unit 11A may include, instead of one lens 15, a plurality of lenses individually provided for each of the plurality of irradiation regions R. Instead of one lens 25, the light receiving unit 21A may include a plurality of lenses individually provided for the plurality of irradiation regions R. In this case, each lens may be disposed at a position facing each optical sensor 23 in a one-to-one relationship in the vertical direction A1.

### Second Embodiment

An observation device of a second embodiment will be described. In the following description of the second embodiment, descriptions of parts that overlap those of the first embodiment will be omitted as appropriate, and parts different from those of the first embodiment will be mainly described.

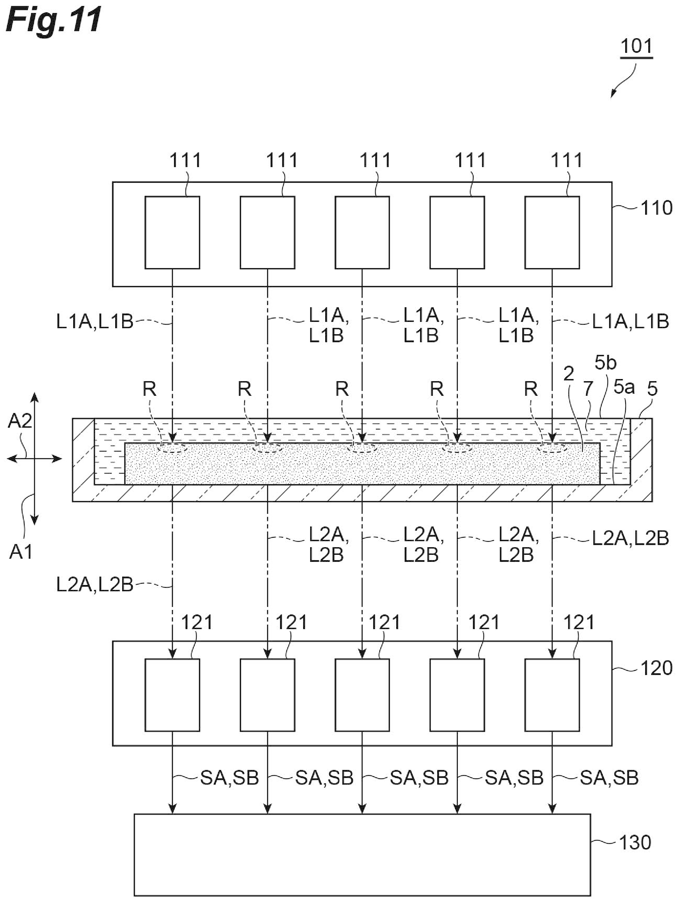

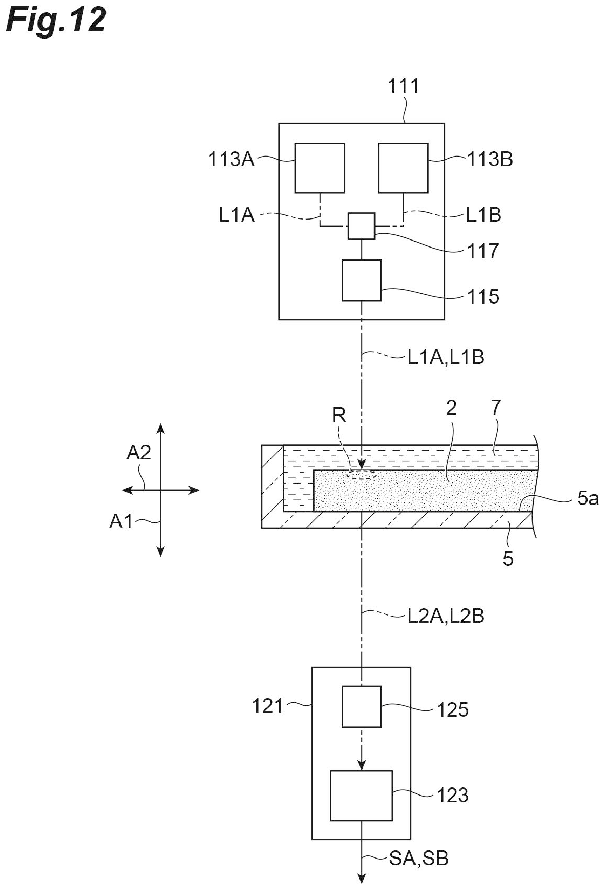

Fig. 11 is a diagram showing a configuration of an observation device 101 of the second embodiment. Fig. 12 is an enlarged view of a part of the observation device 101 of Fig. 11. The observation device 101 of the present embodiment includes an irradiation module 110, a light receiving module 120, and an analysis unit 130. Each of a plurality of light receiving units 121 included in the light receiving module 120 has the same configuration as the light receiving unit 21 of the first embodiment, for example. That is, as shown in Fig. 12, the light receiving unit 121 includes a lens 125 having the same configuration as the lens 25, and an optical sensor 123 having the same configuration as the optical sensor 23.

As in the first embodiment, for example, the plurality of light receiving units 121 are arranged in a planar shape in the lateral direction A2 perpendicular to the vertical direction A1 and the longitudinal direction (depth direction relative to the paper surface in Fig. 1) perpendicular to both the vertical direction A1 and the lateral direction A2. As in the first embodiment, for example, a plurality of irradiation units 111 provided in the irradiation module 110 are also arranged in a planar shape in the lateral direction A2 and the longitudinal direction.

Each of the plurality of irradiation units 111 included in the irradiation module 110 has a different configuration from the irradiation unit 11 of the first embodiment. As shown in Fig. 12, the irradiation unit 111 includes a first light source 113A, a second light source 113B, a lens 115, and a switching unit 117. The lens 115 has the same configuration as the lens 15 in the first embodiment. The first light source 113A and the second light source 113B are disposed above one irradiation region R of the cell population 2. Each of the first light source 113A and the second light source 113B is a laser light source using a laser diode (LD), for example. Each of the first light source 113A and the second light source 113B may be a light source using a semiconductor light emitting element such as a laser diode, a light emitting diode (LED), or a super luminescent diode (SLD), or may be a laser light source using an element other than a semiconductor light emitting element, or a lamp light source.

The first light source 113A emits first irradiation light L1A having a first wavelength included in the ranges of 650 nm to 850 nm and 1060 nm to 1090 nm. The second light source 113B emits second irradiation light L1B having a second wavelength different from the first wavelength. The second wavelength is, for example, a wavelength within the ranges of 650 nm to 850 nm and 1060 nm to 1090 nm, which is different from the first wavelength. Each of the first irradiation light L1A and the second irradiation light L1B may have a plurality of wavelengths. That is, the first irradiation light L1A may have another wavelength different from the first wavelength, and the second irradiation light L1B may have another wavelength different from the second wavelength.

The switching unit 117 is disposed between the first and second light sources 113A and 113B and the lens 115. The switching unit 117 is optically coupled to the first light source 113A, the second light source 113B, and the lens 115 using optical fibers and lenses. The switching unit 117 switches between an optical path of the first irradiation light L1A and an optical path of the second irradiation light L1B so that the first irradiation light L1A from the first light source 113A and the second irradiation light L1B from the second light source 113B are selectively radiated to the irradiation region R of the cell population 2. For example, the switching unit 117 causes the first irradiation light L1A and the second irradiation light L1B to be sequentially radiated to the irradiation region R of the cell population 2. The switching unit 117 may be, for example, an optical switch in which a mirror or prism slides to change the direction in which light travels. The switching unit 117 may be an optical switch that switches a traveling direction of light using a dielectric material with an electro-optic effect, such as LiON3 (lithium nitrate) or LiTaO3 (lithium tantalate).

When the first irradiation light L1A is radiated to the cell population 2, part of the first irradiation light L1A is transmitted through the cell population 2 and is emitted from the cell population 2 as the first emission light L2A. The first emission light L2A has the same wavelength as the first wavelength of the first irradiation light L1A. When the second irradiation light L1B is radiated to the cell population 2, part of the second irradiation light L1B is transmitted through the cell population 2 and is emitted from the cell population 2 as the second emission light L2B. The second emission light L2B has the same second wavelength as the wavelength of the second irradiation light L1B.

The optical sensor 123 selectively receives the first emission light L2A and the second emission light L2B emitted from the cell population 2. For example, the optical sensor 123 receives the first emission light L2A and the second emission light L2B in sequence. The optical sensor 123 outputs, to the analysis unit 130, a first electric signal SA corresponding to an intensity of the first emission light L2A and a second electric signal SB corresponding to an intensity of the second emission light L2B.

The analysis unit 130 quantitatively determines the "number of cells" and the "cell density" of the cell population 2 on the basis of the intensity of the first emission light L2A and the intensity of the second emission light L2B. In general, when light is radiated onto the cell population 2 in the culture medium 7, light absorption by the cell population 2 and the culture medium 7 occurs, and light scattering and reflection from the cell population 2 occurs. Among these phenomena, light scattering and reflection can be broadly divided into Rayleigh scattering, which occurs due to internal molecular structures of the cells forming the cell population 2, and reflection, which occurs due to density fluctuations within the cell population 2.

The Rayleigh scattering intensity generated by the radiation of the light onto the irradiation region R of the cell population 2 depends on the "number of cells" in the irradiation region R. The reflection intensity generated in the irradiation region R depends on the "cell density" in the irradiation region R. The "Rayleigh scattering intensity" indicates an "amount of change in light intensity due to Rayleigh scattering." The Rayleigh scattering intensity depends on a wavelength of light, whereas the reflection intensity is independent of a wavelength of light. For that reason, in the case of observing the state of the cell population 2 using light of a plurality of wavelengths, by utilizing the difference in wavelength dependence, it is possible to determine the wavelength-dependent Rayleigh scattering intensity and the wavelength-independent reflection intensity separately. That is, the "number of cells" can be quantitatively determined on the basis of the Rayleigh scattering intensity, and the "cell density" can be quantitatively determined on the basis of the reflection intensity.

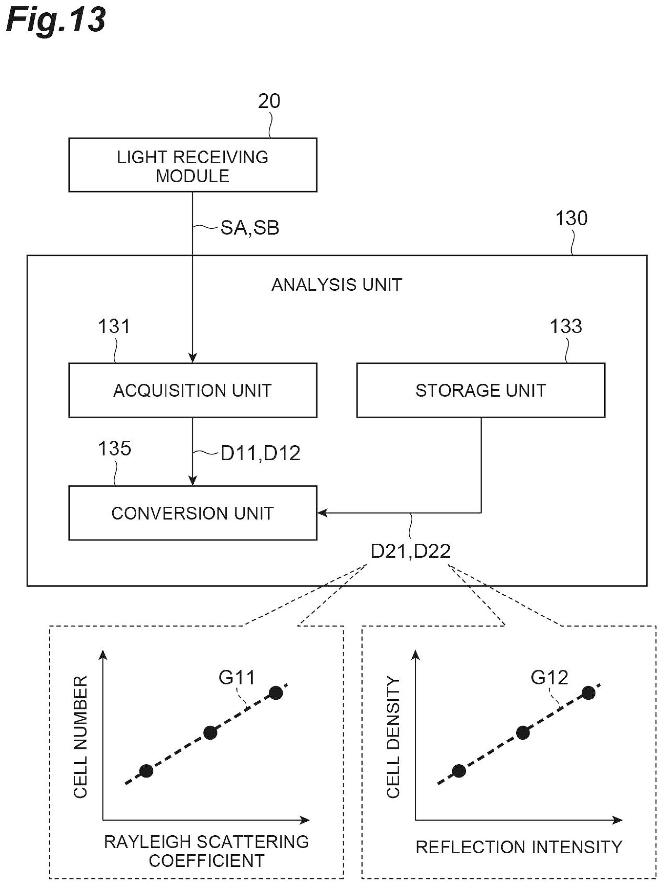

Fig. 13 is a diagram showing a functional configuration of the analysis unit 130. For example, the analysis unit 130 includes, as functional components, an acquisition unit 131, a storage unit 133, and a conversion unit 135. The acquisition unit 131 acquires the intensity of the first emission light L2A indicated by the first electric signal SA and the intensity of the second emission light L2B indicated by the second electric signal SB for each irradiation region R of the cell population 2. The acquisition unit 131 provides the conversion unit 135 with first light intensity information D11 indicating the intensity of the first emission light L2A for each irradiation region R of the cell population 2, and second light intensity information D12 indicating the intensity of the second emission light L2B for each irradiation region R of the cell population 2.

The storage unit 133 stores first conversion information D21 for determining the "number of cells" of the cell population 2 on the basis of the intensity of the first emission light L2A and the intensity of the second emission light L2B, and second conversion information D22 for determining the "cell density" of the cell population 2 on the basis of the intensity of the first emission light L2A and the intensity of the second emission light L2B. The first conversion information D21 includes a first calibration curve G11 showing a relationship between a Rayleigh scattering coefficient, which is obtained from the first light intensity information D11 and the second light intensity information D12, and the "number of cells" of the cell population 2. The "Rayleigh scattering coefficient" is a parameter indicating a relationship between the Rayleigh scattering intensity and the wavelength, which can be determined from the wavelength dependence of the Rayleigh scattering intensity. There is a proportional relationship in which the Rayleigh scattering intensity increases as the Rayleigh scattering coefficient increases. For this reason, the "number of cells" can be quantitatively determined by determining either the Rayleigh scattering intensity or the Rayleigh scattering coefficient. In the present embodiment, a case of determining the "number of cells" using the Rayleigh scattering coefficient is exemplified. The first calibration curve G11 is a graph plotting the relationship between the Rayleigh scattering coefficient and the "number of cells." The second conversion information D22 includes a second calibration curve G12 showing a relationship between the reflection intensity obtained from the first light intensity information D11 and the second light intensity information D12 and the "cell density" of the cell population 2. The second calibration curve G12 is a graph plotting the relationship between the reflection intensity and the "cell density." At least either the first conversion information D21 or the second conversion information D22 may include the intensity of the first irradiation light L1A for determining a first amount of change in light intensity from the first light intensity information D11, or may include the intensity of the second irradiation light L1B for determining a second amount of change in light intensity from the second light intensity information D12.

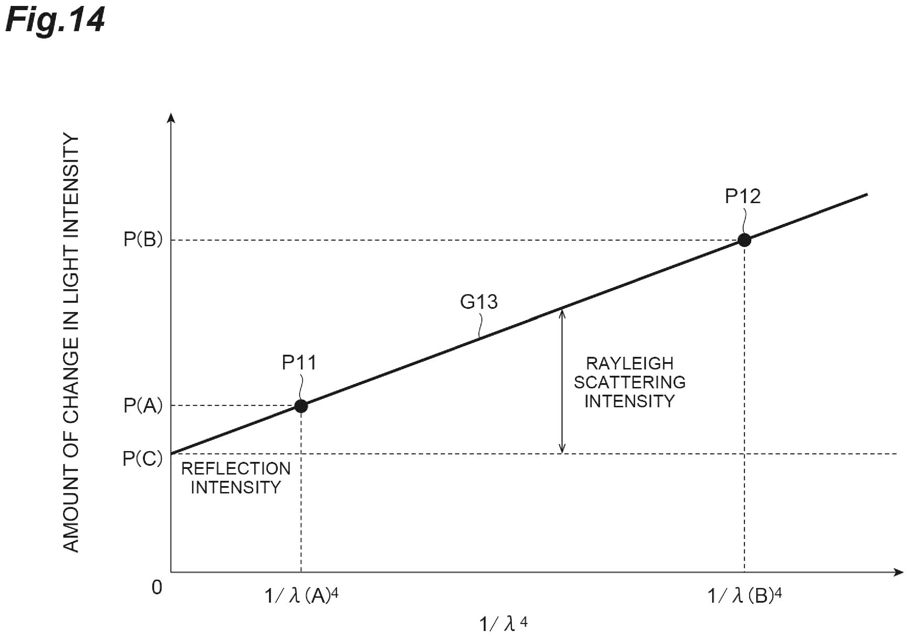

Fig. 14 is a graph for describing how to determine the Rayleigh scattering coefficient and the reflection intensity. A graph G13 shown in Fig. 14 shows a relationship between the amount of change in light intensity and a wavelength λ while there is no absorption and only Rayleigh scattering and reflection. In Fig. 14, the vertical axis represents the amount of change in light intensity, and the horizontal axis represents the reciprocal of the fourth power of the wavelength λ. Fig. 14 shows a plot P11 showing a relationship between a first amount of change in light intensity P(A) and a first wavelength λ(A), and a plot P12 showing a relationship between a second amount of change in light intensity P(B) and a second wavelength λ(B). The first amount of change in light intensity P(A) is a relative ratio of the intensity of the first emission light L2A to the intensity of the first irradiation light L1A, and is defined as -10Log(intensity of first emission light L2A/intensity of first irradiation light L1A). The second amount of change in light intensity P(B) is a relative ratio of the intensity of the second emission light L2B to the intensity of the second irradiation light L1B, and is defined as -10Log(intensity of the second emission light L2B/intensity of the second irradiation light L1B).

In Fig. 14, the amount of change in light intensity is expressed as a sum of the Rayleigh scattering intensity and the reflection intensity. The wavelength-independent reflection intensity is represented by an intercept of a line connecting the plot P11 to the plot P12 (that is, an intensity P(C) indicated by an intersection of the line with the vertical axis of the graph). The wavelength-dependent Rayleigh scattering intensity is represented by a product of a slope of the line connecting the plot P11 to the plot P12 (that is, the Rayleigh scattering coefficient) and the reciprocal of the fourth power of the wavelength λ. Accordingly, the Rayleigh scattering coefficient Pr1 can be determined by the following equation (1), and the reflection intensity Pr2 can be determined by the following equation (2). Once the Rayleigh scattering coefficient Pr1 is determined, it is also possible to determine the Rayleigh scattering intensity using the above relationship.
[Math. 1] $Pr 1 = \left|\frac{P \left(A\right) - P \left(B\right)}{\frac{1}{λ {\left(A\right)}^{4}} - \frac{1}{λ {\left(B\right)}^{4}}}\right|$ (1)
[Math. 2] $Pr 2 = \left|P\left(A\right)-\left(Pr1\times \frac{1}{λ {\left(A\right)}^{4}}\right)\right|$ (2)

As shown in Fig. 13, the storage unit 133 stores in advance the first conversion information D21 including the first calibration curve G11 showing the relationship between the Rayleigh scattering coefficient and the "number of cells" and the second conversion information D22 including the second calibration curve G12 showing the relationship between the reflection intensity and the "cell density." By preparing in advance a sample of the cell population 2 with a "number of cells" and a "cell density" which are already known (or a sample having a "number of cells" and a "cell density" serving as references) and irradiating the sample with the first irradiation light L1A and the second irradiation light L1B using the observation device 101, the first calibration curve G11 showing the relationship between the Rayleigh scattering coefficient and the "number of cells" of the sample, and the second calibration curve G12 showing the relationship between the reflection intensity and the "cell density" can be created in advance.

The conversion unit 135 uses the first light intensity information D11 and the second light intensity information D12 from the acquisition unit 131 and the first conversion information D21 and the second conversion information D22 from the storage unit 133 to determine the "number of cells" and the "cell density" for each irradiation region R of the cell population 2. Specifically, first, the conversion unit 135 calculates the first amount of change in light intensity P(A), which is a relative ratio between the intensity of the first emission light L2A and the intensity of the first irradiation light L1A, and calculates the second amount of change in light intensity P(B), which is a relative ratio between the intensity of the second emission light L2B and the intensity of the second irradiation light L1B. Next, the conversion unit 135 uses the first amount of change in light intensity P(A) and the second amount of change in light intensity P(B) to determine the Rayleigh scattering coefficient Pr1 shown in Equation (1). The conversion unit 135 uses the Rayleigh scattering coefficient Pr1 to determine the reflection intensity Pr2 shown in Equation (2).

After that, the conversion unit 135 converts the Rayleigh scattering coefficient Pr1 into the "number of cells" by referring to the first calibration curve G11. The conversion unit 135 converts the reflection intensity Pr2 into the "cell density" by referring to the second calibration curve G12. Thus, the "number of cells" and the "cell density" for each irradiation region R of the cell population 2 can be determined separately.

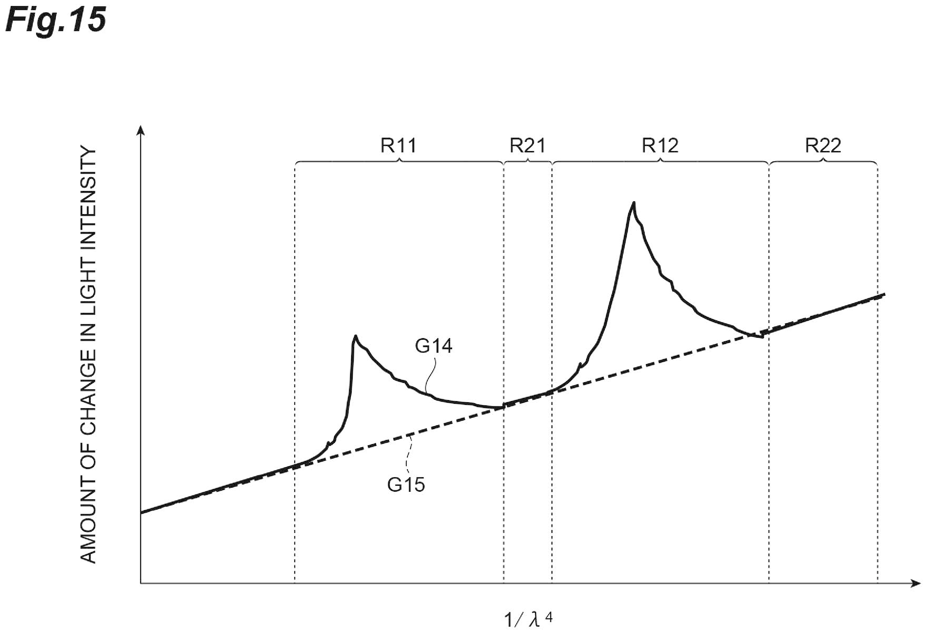

Fig. 15 is a graph for describing wavelength ranges suitable for observing the state of the cell population 2. A graph G14, represented by the solid line in Fig. 15, shows the wavelength dependence of the amount of change in light intensity, which reflects the influence of light absorption by the cells and the culture medium 7. A graph G15, represented by the dotted line in Fig. 15, shows the wavelength dependence of the amount of change in light intensity when there is no influence of light absorption by the cells and the culture medium 7. As shown in Fig. 15, the graph G14 includes wavelength ranges R11 and R12, in which peaks with a steep increase in the amount of change in light intensity exist, and wavelength ranges R21 and R22, in which such peaks do not exist. In the wavelength ranges R11 and R12 in which such peaks exist, it may be difficult to accurately determine the Rayleigh scattering coefficient due to the influence of peaks. Accordingly, in order to accurately determine the Rayleigh scattering coefficient, it is appropriate to use wavelengths included in the wavelength ranges R21 and R22 in which no peaks exist.

In the present embodiment, the first wavelength λ(A) of the first irradiation light L1A and the second wavelength λ(B) of the second irradiation light L1B are both included in the wavelength ranges R2 (see Fig. 6) of 650 nm to 850 nm and 1060 nm to 1090 nm. In the graph G14 in Fig. 15, the wavelengths of 1060 nm to 1090 nm are included in the wavelength range R21, in which no peak exists, and the wavelengths of 650 nm to 850 nm are included in the wavelength range R22, in which no peak exists. Accordingly, in the case of irradiating the irradiation region R of the cell population 2 with the first irradiation light L1A and the second irradiation light L1B described above, the amount of change in light intensity can be obtained without being affected by the absorption of the irradiation light by the cell population 2 and the culture medium 7, and thus it is possible to accurately determine the Rayleigh scattering coefficient on the basis of the amount of change in light intensity.

Effects obtained by the second embodiment described above will be described. As described above, when the cell population 2 is irradiated with light, Rayleigh scattering occurs due to internal molecular structures of the cells forming the cell population 2, and reflection occurs due to density fluctuations within the cell population 2. The Rayleigh scattering intensity depends on the "number of cells." The reflection intensity depends on the "cell density." The Rayleigh scattering intensity is wavelength dependent. The reflection intensity is not wavelength dependent. Therefore, in the case of observing the state of the cell population 2 using light of a plurality of wavelengths, by utilizing the difference in wavelength dependence, it is possible to determine the wavelength-dependent Rayleigh scattering intensity and the wavelength-independent reflection intensity separately. That is, it is possible to quantitatively determine the "number of cells" and the "cell density" separately. Thus, it is possible to observe the state of the cell population 2 in more detail.

As in the present embodiment, the irradiation unit 111 may include the first light source 113A, the second light source 113B, and the switching unit 117. The light receiving unit 121 may include an optical sensor 123. In this case, the intensity of the first emission light L2A and the intensity of the second emission light L2B can be detected separately using one optical sensor 123. As a result, since there is no need to prepare separate optical sensors for detecting the intensity of the first emission light L2A and the second emission light L2B, the configuration of the light receiving unit 121 can be kept from becoming complicated.

The observation device and the observation method of the present disclosure are not limited to the second embodiment described above, and various other modifications are possible.

### <Modified Example 1>

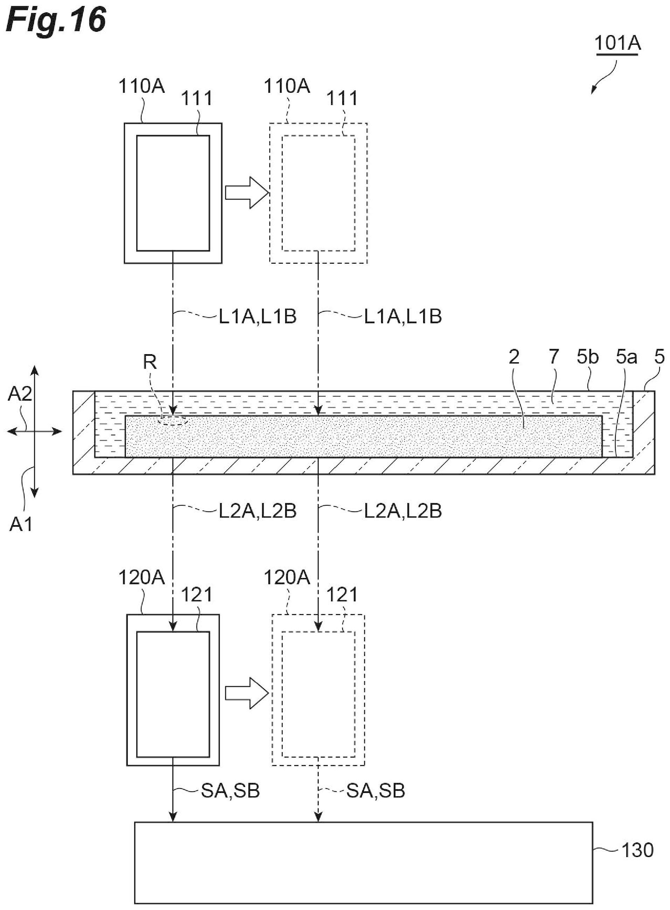

Fig. 16 is a diagram showing a modified example of the observation device 101. As in an observation device 101A shown in Fig. 16, an irradiation module 110A may include only one irradiation unit 111, and a light receiving module 120A may include only one light receiving unit 121. In this case, the irradiation unit 111 is disposed above one of the irradiation regions R of the cell population 2 and irradiates the irradiation region R with the first irradiation light L1A and the second irradiation light L1B. The light receiving unit 121 is disposed at a position opposite to the irradiation unit 111 with the cell population 2 interposed therebetween. That is, the light receiving unit 121 is disposed below the irradiation region R irradiated with the first irradiation light L1A and the second irradiation light L1B, and faces the irradiation unit 111 in the vertical direction A1 with the cell population 2 interposed therebetween.

The irradiation unit 111 and the light receiving unit 121 are configured to be movable in the lateral direction A2 perpendicular to the vertical direction A1 and the longitudinal direction (depth direction relative to the paper surface in Fig. 16) perpendicular to both the vertical direction A1 and the lateral direction A2. The irradiation unit 111 sequentially radiates the first irradiation light L1A and the second irradiation light L1B to each irradiation region R of the cell population 2 while moving in the lateral direction A2 and the longitudinal direction. Similarly, the light receiving unit 121 sequentially receives the first emission light L2A and the second emission light L2B emitted from the cell population 2 as a result of the irradiation of the respective irradiation regions R with the first irradiation light L1A and the second irradiation light L1B while moving in the lateral direction A2 and the longitudinal direction in accordance with the movement of the irradiation unit 111.

The irradiation unit 111 and the light receiving unit 121 may be configured to be movable relative to the container 5 in which the cell population 2 is housed. Accordingly, the irradiation unit 111 and the light receiving unit 121 may be configured to move in the lateral direction A2 and the longitudinal direction while the position of the container 5 remains fixed. Conversely, the container 5 may be configured to move in the lateral direction A2 and the longitudinal direction while the positions of the irradiation unit 111 and the light receiving unit 121 are fixed.

Since the observation device 101A shown in Fig. 16 can also quantitatively determine the "number of cells" and the "cell density" for each irradiation region R of the cell population 2 on the basis of the intensity of the first emission light L2A and the intensity of the second emission light L2B, the same effects as the second embodiment can be obtained. Since the observation device 101A can set each irradiation region R more finely for the cell population 2 in accordance with a movement width of the irradiation unit 111 in the lateral direction A2, it is possible to determine the "number of cells" and the "cell density" in a larger number of irradiation regions R of the cell population 2. That is, it is possible to observe the state of the cell population 2 in more detail.

### <Modified Example 2>

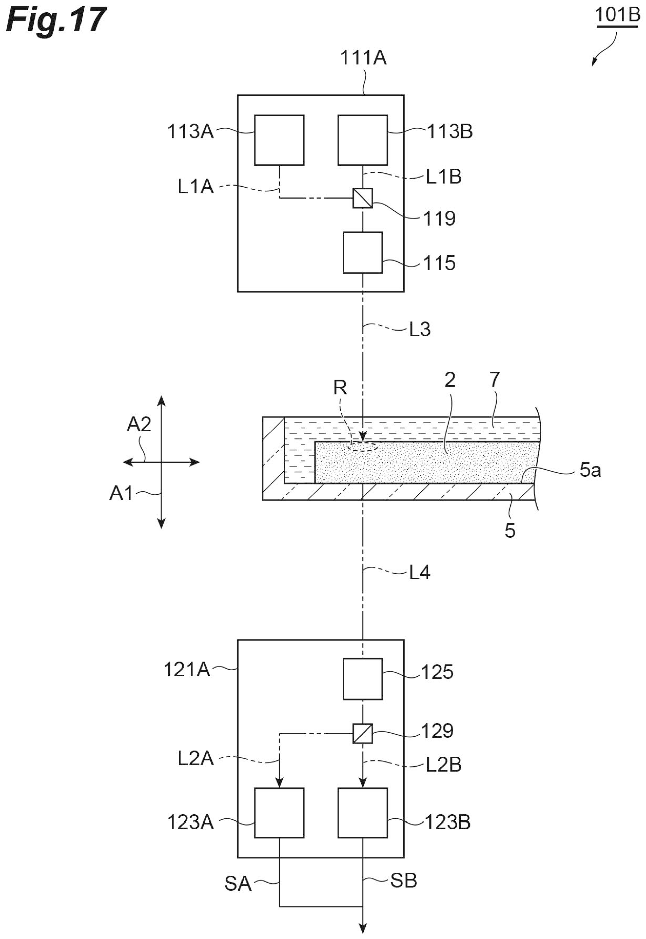

Fig. 17 is a diagram showing a modified example of the observation device 101. As in an observation device 101B shown in Fig. 17, an irradiation unit 111A may include a combining unit 119 instead of the switching unit 117. The combining unit 119 is optically coupled to the first light source 113A, the second light source 113B, and the lens 115 using optical fibers and lenses. The combining unit 119 is disposed between the first and second light sources 113A and 113B and the lens 115. The combining unit 119 combines the first irradiation light L1A from the first light source 113A and the second irradiation light L1B from the second light source 113B to irradiate the irradiation region R of the cell population 2 with the combined irradiation light L3. The combined irradiation light L3 is combined light including the first irradiation light L1A and the second irradiation light L1B, and has the first wavelength λ(A) and the second wavelength λ(B). When the combined irradiation light L3 is radiated to the irradiation region R of the cell population 2, a part of the combined irradiation light L3 passes through the cell population 2 and is emitted from the cell population 2 as the combined emission light L4. The combined emission light L4 has wavelengths corresponding to the first wavelength λ(A) and the second wavelength λ(B) (for example, the same wavelengths as the first wavelength λ(A) and the second wavelength λ(B)). The combining unit 119 is, for example, a dichroic mirror. The combining unit 119 may be an optical fiber multiplexer such as a WDM coupler.

The light receiving unit 121A includes a spectroscopic unit 129, a first optical sensor 123A, and a second optical sensor 123B, instead of the optical sensor 123. The first optical sensor 123A and the second optical sensor 123B are disposed below the cell population 2 and are adjacent to each other in the lateral direction A2. The spectroscopic unit 129 is disposed between the cell population 2 and the first and second optical sensors 123A and 123B. The spectroscopic unit 129 spectrally separates the combined emission light L4 into individual wavelengths. For example, the spectroscopic unit 129 spectrally separates the combined emission light L4 into the first emission light L2A having the first wavelength λ(A) and the second emission light L2B having the second wavelength λ(B). The first optical sensor 123A receives the first emission light L2A separated from the combined emission light L4 by the spectroscopic unit 129 and outputs the first electric signal SA indicating the intensity of the first emission light L2A. The second optical sensor 123B receives the second emission light L2B separated from the combined emission light L4 by the spectroscopic unit 129 and outputs the second electric signal SB indicating the intensity of the second emission light L2B. The spectroscopic unit 129 is, for example, a dichroic mirror. The spectroscopic unit 129 may be an optical fiber type demultiplexer such as a WDM coupler.

Since the observation device 101B shown in Fig. 17 can also quantitatively determine the "number of cells" and the "cell density" for each irradiation region R of the cell population 2 on the basis of the intensity of the first emission light L2A and the intensity of the second emission light L2B, the same effects as the second embodiment can be obtained. With the observation device 101B, the intensity of the first emission light L2A and the intensity of the second emission light L2B can be detected simultaneously using the combined irradiation light L3 and the combined emission light L4, and thus the state of each irradiation region R of the cell population 2 based on the intensity of the first emission light L2A and the intensity of the second emission light L2B can be observed more efficiently in a shorter amount of time.

### <Modified Example 3>

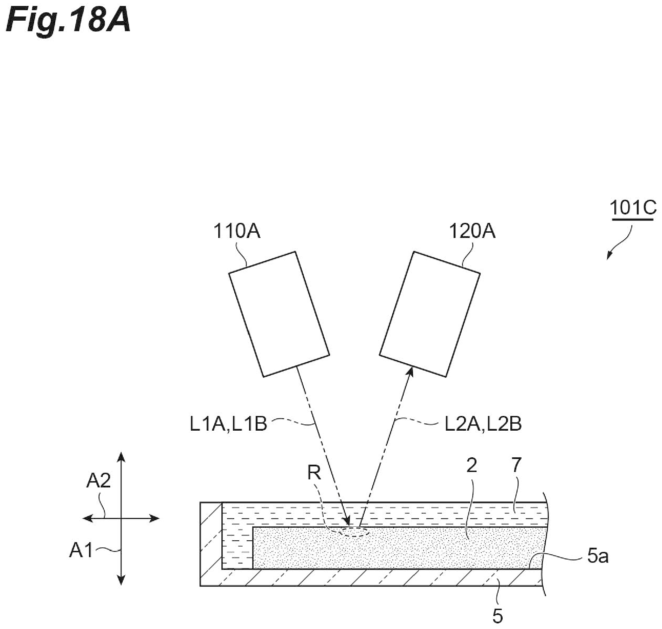

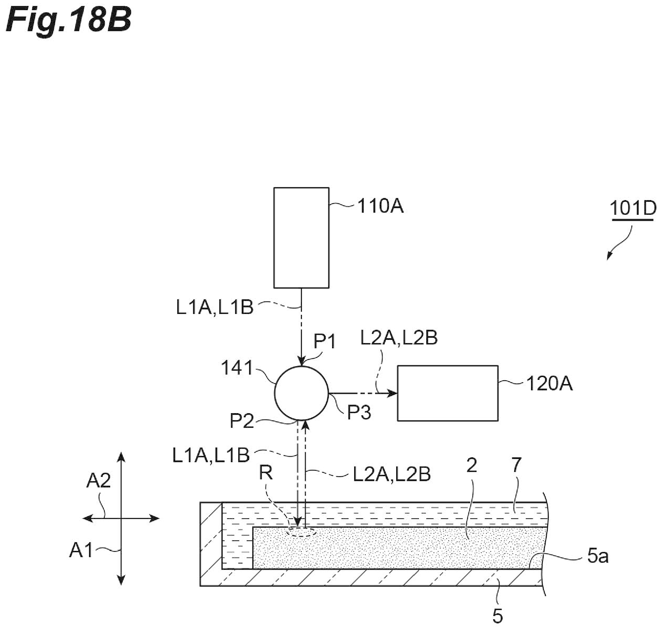

Figs. 18A and 18B are diagrams showing a modified example of the observation device 101. Each of an observation device 101C shown in Fig. 18A and an observation device 101D shown in Fig. 18B includes, for example, the irradiation module 110A and the light receiving module 120A shown in Fig. 16. Each of the observation devices 101C and 101D may include the irradiation module 110 and the light receiving module 120 of the second embodiment. In the observation devices 101C and 101D, the light receiving module 120A is disposed above the cell population 2, similarly to the irradiation module 110A. That is, of two regions sandwiching the cell population 2, the light receiving module 120A is disposed in the same region as the region in which the irradiation module 110A is located.

In the observation device 101C shown in Fig. 18A, the optical axes of the first irradiation light L1A and the second irradiation light L1B (hereinafter simply referred to as "irradiation lights L1A and L1B") emitted from the irradiation module 110A are inclined with respect to the vertical direction A1. Part of the first irradiation light L1A radiated to the irradiation region R of the cell population 2 is reflected on the surface of or inside the cell population 2. The reflection light reflected from the cell population 2 is emitted from the cell population 2 as the first emission light L2A. Part of the second irradiation light L1B radiated to the irradiation region R of the cell population 2 is reflected on the surface of or inside the cell population 2. The reflection light reflected from the cell population 2 is emitted from the cell population 2 as the second emission light L2B. The optical axes of the first emission light L2A and the second emission light L2B are inclined with respect to the vertical direction A1.

The light receiving module 120A is disposed on the optical paths of the emission lights L2A and L2B. The light receiving module 120A is disposed, for example, adjacent to the irradiation module 110A in the lateral direction A2. The light receiving module 120A receives the first emission light L2A and the second emission light L2B (hereinafter simply referred to as "emission lights L2A and L2B") and detects the intensities of the emission lights L2A and L2B. Similarly to the observation device 101 of the second embodiment, the observation device 101C determines the Rayleigh scattering coefficient and the reflection intensity on the basis of the intensities of the emission lights L2A and L2B.

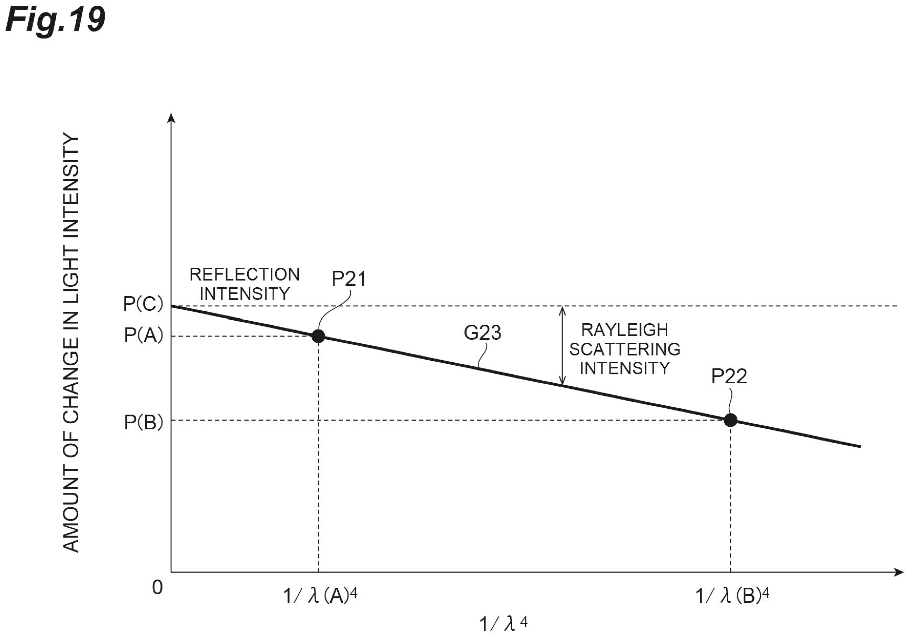

Fig. 19 is a graph for describing how to determine the Rayleigh scattering coefficient and the reflection intensity. In Fig. 19, the vertical axis represents the amount of change in light intensity and the horizontal axis represents the reciprocal of the fourth power of the wavelength λ. Fig. 19 shows a plot P21 showing a relationship between the first amount of change in light intensity P(A) and the first wavelength λ(A) of the first emission light L2A, and a plot P22 showing a relationship between the second amount of change in light intensity P(B) and the second wavelength λ(B) of the second emission light L2B. The first amount of change in light intensity P(A) is a relative ratio of the intensity of the first emission light L2A to the intensity of the first irradiation light L1A, and is defined as -10Log(intensity of first emission light L2A/intensity of first irradiation light L1A). The second amount of change in light intensity P(B) is a relative ratio of the intensity of the second emission light L2B to the intensity of the second irradiation light L1B, and is defined as -10Log(intensity of the second emission light L2B/intensity of the second irradiation light L1B).

In a graph G23 in Fig. 19, the amount of change in light intensity is expressed as a sum of the Rayleigh scattering intensity and the reflection intensity. The wavelength-independent reflection intensity is represented by an intercept of a line connecting the plot P21 to the plot P22 (that is, the intensity P(C) indicated by an intersection of the line and the vertical axis of the graph). The wavelength-dependent Rayleigh scattering intensity is expressed as a product of a slope of the line connecting the plot P21 to the plot P22 (that is, the Rayleigh scattering coefficient) and the reciprocal of the fourth power of the wavelength λ. Accordingly, the Rayleigh scattering coefficient Pr1 can be determined by the above-described equation (1). The following equation (3) can be used to determine the reflection intensity Pr3. Then, by converting the Rayleigh scattering coefficient Pr1 to the "number of cells" and the reflection intensity Pr3 to "cell density," each of the "number of cells" and the "cell density" can be quantitatively determined. Accordingly, in the observation device 101C, the same effects as the second embodiment can also be obtained.
[Math. 3] $Pr 3 = \left|P\left(A\right)+\left(Pr1\times \frac{1}{λ {\left(A\right)}^{4}}\right)\right|$ (3)

In the observation device 101D shown in Fig. 18B, a circulator 141 is disposed above the irradiation region R of the cell population 2. The irradiation module 110A is disposed further above the circulator 141. The light receiving module 120A is disposed at a position away from the circulator 141 in the lateral direction A2. The circulator 141 is disposed at an intersection of the optical axes of the irradiation lights L1A and L1B emitted from the irradiation module 110A and the optical axes of the emission lights L2A and L2B incident on the light receiving module 120A.

The circulator 141 is an optical component for separating two beams of light traveling in opposite directions from each other and has three ports P1, P2, and P3 for the light. The irradiation lights L1A and L1B emitted from the irradiation module 110A are incident on the port P1 of the circulator 141 and are emitted from the port P2 to the irradiation region R of the cell population 2. The emission lights L2A and L2B emitted from the cell population 2 toward the port P2 are emitted from the port P3 to the light receiving module 120A. Since the observation device 101D can also quantitatively determine the "number of cells" and the "cell density" of the cell population 2 for each irradiation region R of the cell population 2 on the basis of the intensities of the emission lights L2A and L2B from the cell population 2, the same effects as the second embodiment can be obtained.

### <Modified Example 4>

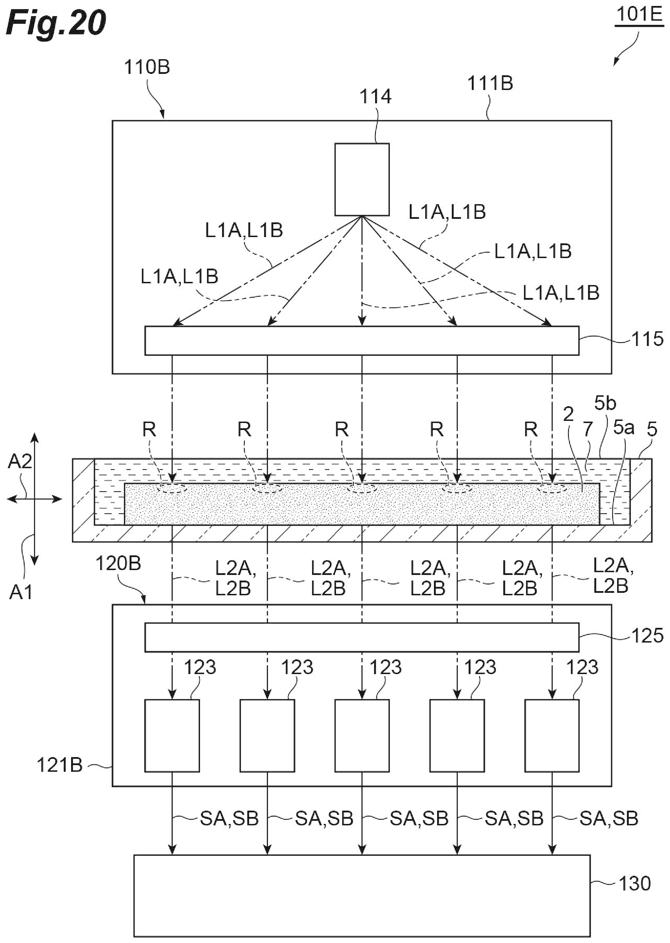

Fig. 20 is a diagram showing a modified example of the observation device 101. As in an observation device 101E shown in Fig. 20, an irradiation module 110B may include only one irradiation unit 111B, and a light receiving module 120B may include only one light receiving unit 121B. In the example shown in Fig. 20, the irradiation unit 111B includes one light source unit 114 and one lens 115. The light source unit 114 is configured to be able to uniformly irradiate a wide area with the irradiation lights L1A and L1B. The light source unit 114 is disposed above the cell population 2 and uniformly radiates the irradiation lights L1A and L1B over a wide area extending two-dimensionally when viewed from above. A range of the irradiation lights L1A and L1B radiated from the light source unit 114 may be a region extending in a planar shape in the lateral direction A2 and the longitudinal direction (depth direction relative to the paper surface in Fig. 20). The light source unit 114 radiates the irradiation light L1A or the irradiation light L1B simultaneously and uniformly to a plurality of irradiation regions R arranged in the lateral direction A2 and the longitudinal direction, which are set in the cell population 2.

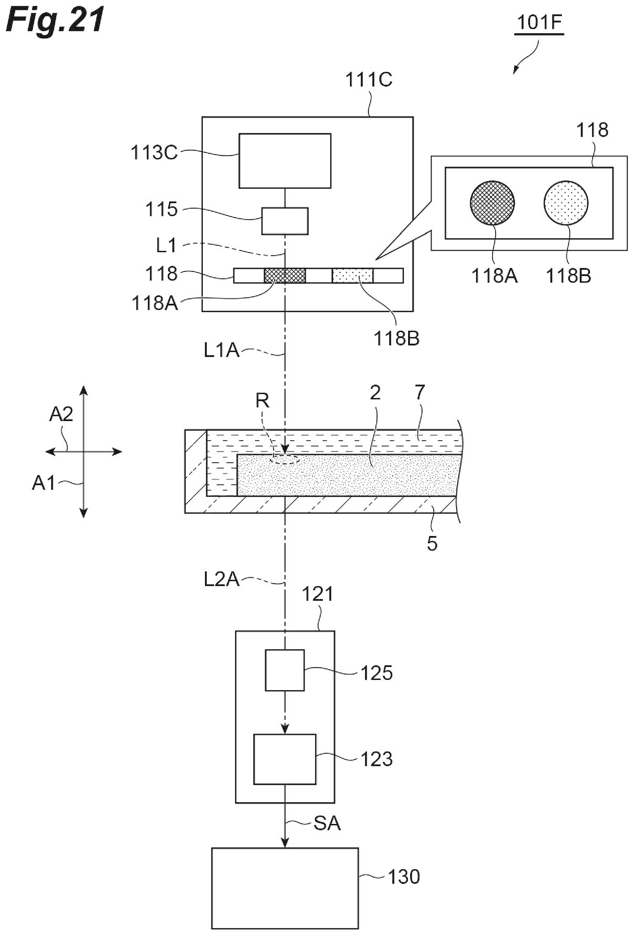

The light source unit 114 may have, for example, a configuration including the first light source 113A, the second light source 113B, and the switching unit 117 (see Fig. 12), or a configuration including the first light source 113A, the second light source 113B, and the combining unit 119 (see Fig. 17). Alternatively, the light source unit 114 may have a configuration including a light source 113C, the lens 115, and a filter switching unit 118 (see Fig. 21 described later.). The light source unit 114 is not limited to these configurations, and may have another configuration as long as it can radiate the irradiation lights L1A and L1B over a wide area extending two-dimensionally when viewed from above.

The lens 115 is disposed on the optical paths of the irradiation lights L1A and L1B between the light source unit 114 and the cell population 2. The lens 115 is, for example, a collimating lens that collimates the irradiation lights L1A and L1B emitted over a wide area from the light source unit 114. The lens 115 extends in a planar shape in the lateral direction A2 and the longitudinal direction so as to face the light source unit 114 in the vertical direction A1 and so as to face all irradiation regions R set in the cell population 2 in the vertical direction A1. The irradiation lights L1A and L1B emitted from the light source unit 114 in a diverging manner are collimated by the lens 115 and are radiated to each irradiation region R of the cell population 2. Part of the irradiation light L1A passes through the cell population 2 and is emitted as the emission light L2A, and part of the irradiation light L1B passes through the cell population 2 and is emitted as the emission light L2B.

The light receiving unit 121B includes a plurality of optical sensors 123 and one lens 125. The plurality of optical sensors 123 are respectively disposed below the plurality of irradiation regions R of the cell population 2. Each optical sensor 123 selectively receives the emission lights L2A and L2B emitted from the cell population 2 as a result of irradiating the irradiation region R of the cell population 2 with the irradiation lights L1A and L1B.

The lens 125 is disposed on the optical paths of the plurality of emission lights L2A and L2B between the cell population 2 and the plurality of optical sensors 123. The lens 125 is, for example, an objective lens. The lens 125 extends in a planar shape in the lateral direction A2 and the longitudinal direction so that it faces all irradiation regions R set in the cell population 2 in the vertical direction A1 and faces all optical sensors 123 in the vertical direction A1. The lens 125 faces the lens 115 in the vertical direction A1 with the cell population 2 interposed therebetween. Each of the emission lights L2A and L2B emitted from the cell population 2 is incident on each optical sensor 123 through the lens 125. Each optical sensor 123 selectively receives the emission lights L2A and L2B and outputs the electric signals SA and SB corresponding to the intensities of the emission lights L2A and L2B.

Since the observation device 101E shown in Fig. 20 can also quantitatively determine the "number of cells" and the "cell density" for each irradiation region R of the cell population 2 on the basis of the intensity of each of the emission lights L2A and L2B from the cell population 2, the same effects as the second embodiment can be obtained. The observation device 101E can irradiate each irradiation region R with the irradiation lights L1A and L1B using one light source unit 114, and thus as compared to the case of irradiating each irradiation region R with the irradiation lights L1A and L1B using a plurality of light source units, the device configuration can be simplified by reducing the number of light source units.

In the observation device 101E, the irradiation unit 111B may include a plurality of lenses individually provided for each of the plurality of irradiation regions R, instead of one lens 115. Instead of one lens 125, the light receiving unit 121B may include a plurality of lenses individually provided for each of the plurality of irradiation regions R. In this case, each lens is disposed at a position facing each optical sensor 123 in a one-to-one relationship in the vertical direction A1.

### <Modified Example 5>

Fig. 21 is a diagram showing a modified example of the observation device 101. As shown in Fig. 21, an observation device 101F may include an irradiation unit 111C instead of the irradiation unit 111. The irradiation unit 111C includes the light source 113C, the lens 115, and the filter switching unit 118. The light source 113C is a halogen lamp (lamp light source) that emits the irradiation light L1 including a plurality of wavelengths. At least one wavelength included in the irradiation light L1 is included in the ranges of 650 nm to 850 nm and 1060 nm to 1090 nm. Other wavelengths included in the irradiation light L1 may be included in the ranges of 650 nm to 850 nm and 1060 nm to 1090 nm, or may be outside the ranges of 650 nm to 850 nm and 1060 nm to 1090 nm.

The lens 115 is optically coupled to the light source 113C using an optical fiber such as a bundle fiber. The filter switching unit 118 is disposed on the optical path of the irradiation light L1 between the lens 115 and the cell population 2. The filter switching unit 118 holds a first filter 118A and a second filter 118B, and disposes either the first filter 118A or the second filter 118B on the optical path of the irradiation light L1. The first filter 118A is a bandpass filter that transmits only the first wavelength λ(A) of the plurality of wavelengths contained in the irradiation light L1. The second filter 118B is a bandpass filter that transmits only the second wavelength λ(B) of the plurality of wavelengths contained in the irradiation light L1. As shown in the upper right portion of Fig. 21, when the filter switching unit 118 is viewed in the vertical direction A1, each of the first filter 118A and the second filter 118B is, for example, circular.

As shown in Fig. 21, when the filter switching unit 118 disposes the first filter 118A on the optical path of the irradiation light L1, the first irradiation light L1A having the first wavelength λ(A) transmitted through the first filter 118A is radiated to the cell population 2. When the filter switching unit 118 disposes the second filter 118B on the optical path of the irradiation light L1, the second irradiation light L1B having the second wavelength λ(B) transmitted through the second filter 118B is radiated to the cell population 2. The filter switching unit 118 swaps a position of the first filter 118A and a position of the second filter 118B so that the first filter 118A or the second filter 118B is disposed on the optical path of the irradiation light L1 between the light source 113C and the cell population 2. Thus, the filter switching unit 118 selectively irradiates the cell population 2 with the first irradiation light L1A and the second irradiation light L1B.

When the first irradiation light L1A is radiated to the cell population 2, part of the first irradiation light L1A is transmitted through the cell population 2 and is emitted from the cell population 2 as the first emission light L2A. When the second irradiation light L1B is radiated to the cell population 2, part of the second irradiation light L1B is transmitted through the cell population 2 and is emitted from the cell population 2 as the second emission light L2B. The light receiving unit 121 selectively receives the first emission light L2A and the second emission light L2B emitted from the cell population 2. The light receiving unit 121 outputs the first electric signal SA corresponding to the intensity of the first emission light L2A and the second electric signal SB corresponding to the intensity of the second emission light L2B to the analysis unit 130.

Since the observation device 101F shown in Fig. 21 can also quantitatively determine the "number of cells" and the "cell density" for each irradiation region R of the cell population 2 on the basis of the intensity of the first emission light L2A and the intensity of the second emission light L2B, the same effects as the second embodiment can be obtained. As in the observation device 101F, by using the light source 113C, which is an inexpensive lamp light source, the cost of the observation device 101F can be reduced as compared to when an expensive laser light source is used.

The present disclosure is not limited to the above-described embodiments and modified examples, and various other modifications are possible. For example, each of the embodiments and each of the modified examples described above may be combined with each other to the extent that they are consistent, depending on the required purpose and effect. In each of the embodiments and each of the modified examples described above, one cell population is contained in a container, but a plurality of cell populations may be contained in a container. The cell population may be suspended in the culture medium. In each of the embodiments and each of the modified examples described above, a case in which the irradiation module is disposed above the cell population and the light receiving module is disposed below the cell population has been mainly described, but the irradiation module and the light receiving module may be reversed. That is, the irradiation module may be disposed below the cell population, and the light receiving module may be disposed above the cell population.

In each of the embodiments and each of the modified examples described above, a case in which the irradiation light radiated to the cell population includes at least one wavelength included in the ranges of 650 nm to 850 nm and 1060 nm to 1090 nm has been described. However, all wavelengths of the irradiation light may be included in the ranges of 650 nm to 850 nm and 1060 nm to 1090 nm. That is, the irradiation light may have only wavelengths in the ranges of 650 nm to 850 nm and 1060 nm to 1090 nm. In this case, a decrease in measurement accuracy due to mixing of wavelengths outside the ranges of 650 nm to 850 nm and 1060 nm to 1090 nm into the irradiation light can be avoided. The analysis unit may handle only information (irradiation light intensity or emission light intensity) obtained from wavelengths within the ranges of 650 nm to 850 nm and 1060 nm to 1090 nm.

### Reference Signs List

1, 1A, 1B, 1C, 1D, 101, 101A, 101B, 101C, 101D, 101E, 101F Observation device
2 Cell population
5 Container
5a Bottom surface
5b Opening
7 Culture medium
10, 10A, 10B, 110, 110A, 110B Irradiation module
11, 11A, 111, 111A, 111B, 111C Irradiation unit
13 Light source (laser light source)
15, 25, 115, 125 Lens
20, 20A, 20B, 120, 120A, 120B Light receiving module
21, 21A, 121, 121A, 121B Light receiving unit
23, 123 Optical sensor
30, 130 Analysis unit
31, 131 Acquisition unit
33, 133 Storage unit
35, 135 Conversion unit
41, 141 Circulator
113A First light source
113B Second light source
113C Light source
114 Light source unit
117 Switching unit
118 Filter switching unit
118A First filter
118B Second filter
119 Combining unit
123A First optical sensor
123B Second optical sensor
129 Spectroscopic unit
301 Processor
302 Main storage device
303 Auxiliary storage device
304 Input device
305 Output device
A1 Vertical direction
A2 Lateral direction
D1 Light intensity information
D2 Conversion information
D11 First light intensity information
D12 Second light intensity information
D21 First conversion information
D22 Second conversion information
G1 Calibration curve
G11 First calibration curve
G12 Second calibration curve
L1 Irradiation light
L1A First irradiation light
L1B Second irradiation light
L2 Emission light
L2A First emission light
L2B Second emission light
L3 Combined irradiation light
L4 Combined emission light
P1, P2, P3 Port
P(A) First amount of change in light intensity
P(B) Second amount of change in light intensity
Pr1 Rayleigh scattering coefficient
Pr2, Pr3 Reflection intensity
R Irradiation region
R1, R2, R11, R21, R22 Wavelength range
λ Wavelength
λ(A) First wavelength
λ(B) Second wavelength
S Electric signal
SA First electric signal
SB Second electric signal

## Claims

1. An observation device configured to observe a state of a cell population configured of a plurality of cells, comprising:
at least one irradiation unit that is disposed at a position facing the cell population and irradiates at least one irradiation region set in the cell population with irradiation light having at least one wavelength included in the ranges of 650 nm to 850 nm and 1060 nm to 1090 nm;
at least one light receiving unit that is disposed at a position facing the cell population and receives emission light emitted from the cell population as a result of irradiating the irradiation region with the irradiation light; and
an analysis unit that is communicatively connected to the light receiving unit and determines a parameter including at least one of the number of cells and a cell density of the cell population in the irradiation region on the basis of an intensity of the emission light.

2. The observation device according to claim 1, wherein the irradiation unit includes a light source using a semiconductor light emitting element that emits light having one wavelength included in the ranges of 650 nm to 850 nm and 1060 nm to 1090 nm as the irradiation light.

3. The observation device according to claim 1, wherein the light receiving unit is disposed at a position opposite to the irradiation unit with the cell population interposed therebetween, and receives part of the irradiation light emitted from the irradiation unit and transmitted through the cell population as the emission light.

4. The observation device according to any one of claims 1 to 3,
wherein the irradiation unit includes one or more light sources that emit the irradiation light, and radiates the irradiation light to each of a plurality of the irradiation regions set in the cell population, and
the light receiving unit includes one or more optical sensors on which the emission light is incident, and receives a plurality of the irradiation lights transmitted through the cell population as a plurality of the emission lights.

5. The observation device according to any one of claims 1 to 4,
wherein the irradiation unit irradiates the irradiation region of the cell population with first irradiation light having a first wavelength included in the ranges of 650 nm to 850 nm and 1060 nm to 1090 nm, and second irradiation light having a second wavelength included in the ranges of 650 nm to 850 nm and 1060 nm to 1090 nm, which is different from the first wavelength,
the light receiving unit receives the first emission light and the second emission light, which have different wavelengths from each other, and which are emitted from the cell population as a result of radiating the first irradiation light and the second irradiation light, and
the analysis unit determines each of the number of cells and the cell density in the irradiation region on the basis of an intensity of the first emission light and an intensity of the second emission light.

6. The observation device according to claim 5,
wherein the irradiation unit includes
a first light source configured to emit the first irradiation light,
a second light source configured to emit the second irradiation light, and
a switching unit configured to switch between an optical path of the first irradiation light and an optical path of the second irradiation light so that the first irradiation light and the second irradiation light are selectively radiated to the irradiation region of the cell population,
and
the light receiving unit includes
an optical sensor configured to selectively receive the first emission light and the second emission light emitted from the cell population.

7. The observation device according to claim 5,
wherein the irradiation unit includes
a first light source configured to emit the first irradiation light,
a second light source configured to emit the second irradiation light, and
a combining unit configured to combine the first irradiation light and the second irradiation light to generate combined irradiation light and irradiate the irradiation region of the cell population with the combined irradiation light,
and
the light receiving unit includes
a spectroscopic unit configured to spectrally separate the combined emission light emitted from the cell population as a result of radiating the combined irradiation light into each wavelength,
a first optical sensor configured to detect an intensity of first emission light separated from the combined emission light, and
a second optical sensor configured to detect an intensity of second emission light separated from the combined emission light.

8. The observation device according to claim 5,
wherein the irradiation unit includes
a light source configured to emit light including the first irradiation light and the second irradiation light, and
a filter switching unit that includes a first filter configured to transmit only the first irradiation light and a second filter configured to transmit only the second irradiation light, and switches between a position of the first filter and a position of the second filter so that the first filter or the second filter is disposed on an optical path of the light between the light source and the cell population,
and
the light receiving unit includes
an optical sensor configured to selectively receive the first emission light and the second emission light emitted from the cell population.

9. An observation method configured to observe a state of a cell population configured of a plurality of cells, comprising the steps of:
irradiating at least one irradiation region set in the cell population with irradiation light having at least one wavelength included in the ranges of 650 nm to 850 nm and 1060 nm to 1090 nm;
receiving emission light emitted from the cell population as a result of irradiating the irradiation region with the irradiation light; and
determining a parameter including at least one of the number of cells and a cell density of the cell population in the irradiation region on the basis of an intensity of the emission light.

10. The observation method according to claim 9,
wherein in the step of irradiating the irradiation region,
first irradiation light having a first wavelength included in the ranges of 650 nm to 850 nm and 1060 nm to 1090 nm and second irradiation light having a second wavelength included in the ranges of 650 nm to 850 nm and 1060 nm to 1090 nm, which is different from the first wavelength, are radiated to the irradiation region,
in the step of receiving the emission light,
first emission light and second emission light, which have different wavelengths from each other, and which are emitted from the cell population as a result of radiating the first irradiation light and the second irradiation light, are received, and
in the step of determining the parameter,
each of the number of cells and the cell density in the irradiation region is determined on the basis of an intensity of the first emission light and an intensity of the second emission light.
